# EUROPÄISCHE PATENTSCHRIFT

(11) **EP 1 060 176 B1**
(45) Veröffentlichungstag und Bekanntmachung des Hinweises auf die Patenterteilung: **19.05.2004**
(21) Anmeldenummer: 98955432.4
(22) Anmeldetag: 12.10.1998
(51) Int. Cl.: C07D 405/06, A01N 43/653

(54) **OXIRANYL-TRIAZOLINTHIONE UND IHRE VERWENDUNG ALS MIKROBIZIDE**
OXYRANYLE-TRIAZOLINE THIONES AND THEIR USE AS MICROBICIDES
OXYRANYLE-TRIAZOLINE THIONES ET LEUR UTILISATION COMME MICROBICIDES

(30) Priorität: 24.10.1997 DE 19746993; 28.05.1998 DE 19823861
(43) Veröffentlichungstag der Anmeldung: 20.12.2000
(73) Patentinhaber: Bayer CropScience AG, 40789 Monheim (DE)
(72) Erfinder: HILLEBRAND, Stefan, D-41462 Neuss (DE); JAUTELAT, Manfred, D-51399 Burscheid (DE); MAULER-MACHNIK, Astrid, D-42799 Leichlingen (DE); STENZEL, Klaus, D-40595 Düsseldorf (DE); KUGLER, Martin, D-42799 Leichlingen (DE); EXNER, Otto, D-40878 Ratingen (DE)
(86) Internationale Anmeldenummer: PCT/EP1998/006449
(87) Internationale Veröffentlichungsnummer: WO 1999/021853

(56) Entgegenhaltungen:
- EP-A- 0 548 025
- WO-A-87/06430
- WO-A-96/38440
- DE-A- 19 520 095
- DE-A- 19 617 461

## Beschreibung

Die vorliegende Erfindung betrifft neue Oxiranyl-triazolinthione, ein Verfahren zu deren Herstellung und deren Verwendung als Mikrobizide.

Es ist bereits bekannt geworden, daß zahlreiche Azolylmethyl-oxiran-Derivate fungizide Eigenschaften besitzen (vgl. EP-A 0 094 564, EP-A 0 196 038 und WO-A 96-38 440). So läßt sich z.B. 3-(2-Chlor-phenyl)-2-(4-fluor-phenyl)-2-[(4,5-dihydro-5-thiono-1,2,4-triazol-1-yl)-methyl]-oxiran zur Bekämpfung von Pilzen verwenden. Die Wirksamkeit dieses Stoffes ist gut, läßt aber bei niedrigen Aufwandmengen in manchen Fällen zu wünschen übrig.

Es wurden nun neue Oxiranyl-triazolinthione der Formel 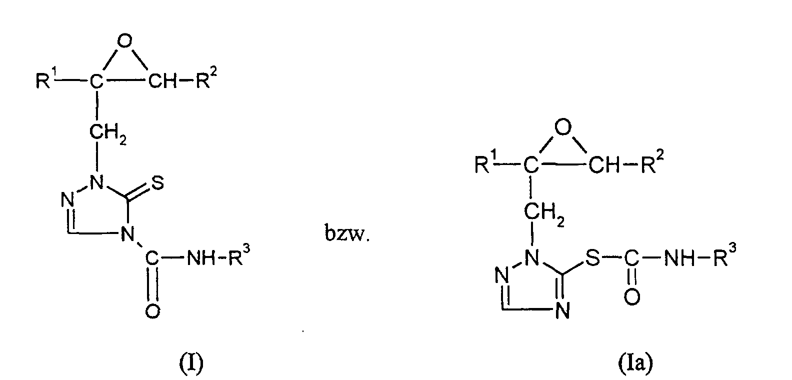 worin
- R¹: für Alkyl mit 1 bis 4 Kohlenstoffatomen, Halogenalkyl mit 1 bis 4 Kohlenstoffatomen und 1 bis 5 Halogenatomen, gegebenenfalls durch Halogen substituiertes Cycloalkyl mit 3 bis 7 Kohlenstoffatomen, Naphthyl oder gegebenenfalls einfach bis dreifach, gleichartig oder verschieden durch Halogen, Nitro, Phenyl, Phenoxy, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen und/oder Halogenalkylthio mit 1 bis 4 Kohlenstoffatomen und 1 bis 5 Halogenatomen substituiertes Phenyl steht,
- R²: für gegebenenfalls einfach bis dreifach, gleichartig oder verschieden durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen und/oder Halogenalkylthio mit 1 bis 4 Kohlenstoffatomen und 1 bis 5 Halogenatomen substituiertes Phenyl steht und
- R³: für Alkoxyalkyl, Isopropyl oder n-Dodecyl steht,
gefunden.

Die erfindungsgemäßen Stoffe enthalten zwei asymmetrisch substituierte Kohlenstoffatome und können deshalb in Form von Diastereomeren oder Enantiomeren anfallen. Die vorliegende Erfindung betrifft sowohl die einzelnen Isomeren als auch deren Gemische.

Weiterhin wurde gefunden, daß man Oxiranyl-triazolinthione der Formel (I) bzw. (Ia) erhält, wenn man Oxiran-Derivate der Formel 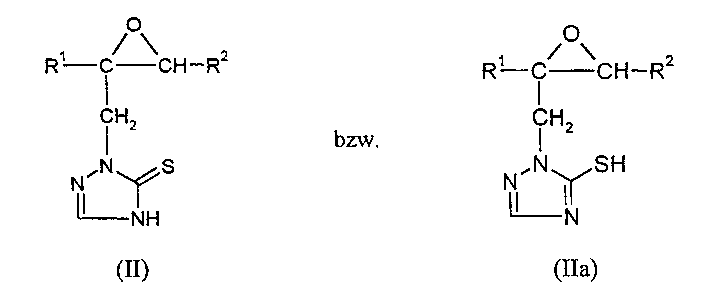 worin
R¹ und R² die oben angegebenen Bedeutungen haben,
mit Isocyanaten der Formel

R³-NCO (III),

in welcher
- R³: die oben angegebene Bedeutung hat,
gegebenenfalls in Gegenwart eines Katalysators und gegebenenfalls in Gegenwart eines Verdünnungsmittels umsetzt.

Schließlich wurde gefunden, daß die neuen Oxiranyl-triazolinthione der Formel (I) bzw. (Ia) sehr gute mikrobizide Eigenschaften aufweisen und sich sowohl im Pflanzenschutz als auch im Materialschutz zur Bekämpfung unerwünschter Mikroorganismen verwenden lassen.

Überraschenderweise besitzen die erfindungsgemäßen Oxiranyl-triazolinthione der Formel (I) bzw. (Ia) eine bessere mikrobizide Wirksamkeit, insbesondere fungizide Wirksamkeit als die konstitutionell ähnlichsten, vorbekannten Verbindungen gleicher Wirkungsrichtung. So übertreffen die erfindungsgemäßen Stoffe das 3-(2-Chlorphenyl)-2-(4-fluor-phenyl)-2-[(4,5-dihydro-5-thiono-1,2,4-triazol-1-yl)-methyl]-oxiran bezüglich der fungiziden Eigenschaften.

Die erfindungsgemäßen Oxiranyl-triazolinthione sind durch die Formel (I) bzw. (Ia) allgemein definiert.
- R¹: steht vorzugsweise für Methyl, Ethyl, n-Propyl, Isopropyl, n-Butyl, sek.-Butyl, iso-Butyl, tert.-Butyl, Fluor-tert.-butyl, Difluor-tert.-butyl, gegebenenfalls einfach bis dreifach, gleichartig oder verschieden durch Fluor, Chlor und/oder Brom substituiertes Cycloalkyl mit 3 bis 6 Kohlenstoffatomen, für Naphthyl oder für Phenyl, das einfach bis dreifach, gleichartig oder verschieden substituiert sein kann durch Fluor, Chlor, Brom, Nitro, Phenyl, Phenoxy, Methyl, Ethyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio.
- R²: steht vorzugsweise für Phenyl, das einfach bis dreifach, gleichartig oder verschieden substituiert sein kann durch Fluor, Chlor, Brom, Methyl, Ethyl, Isopropyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio.
- R³: steht vorzugsweise für Alkoxyalkyl mit 1 bis 6 Kohlenstoffatomen im Alkoxyteil und 1 bis 6 Kohlenstoffatomen im Alkylteil sowie für Isopropyl oder für n-Dodecyl.

Besonders bevorzugt sind Oxiranyl-triazolinthione der Formel (I) bzw. (Ia), worin
- R¹: für Phenyl steht, das einfach bis dreifach, gleichartig oder verschieden substituiert sein kann durch Fluor, Chlor, Brom, Nitro, Phenyl, Phenoxy, Methyl, Ethyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio,
- R²: für Phenyl steht, das einfach bis dreifach, gleichartig oder verschieden substituiert sein kann durch Fluor, Chlor, Brom, Methyl, Ethyl, Isopropyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio,
und
- R³: für Alkoxyalkyl mit 1 bis 4 Kohlenstoffatomen im Alkoxyteil und 1 bis 4 Kohlenstoffatomen im Alkylteil steht sowie für Isopropyl oder n-Dodecyl steht.

Die genannten Substituenten-Bedeutungen können in beliebiger Weise untereinander kombiniert werden. Außerdem können auch einzelne Definitionen entfallen.

Die bei der Herstellung der erfindungsgemäßen Stoffe als Ausgangssubstanzen benötigten Oxiran-Derivate können in der "Thiono"-Form der Formel 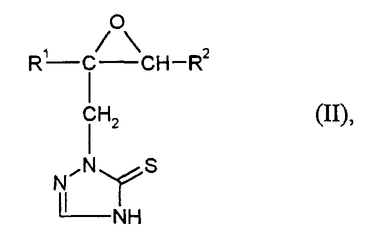 oder in der tautomeren "Mercapto"-Form der Formel 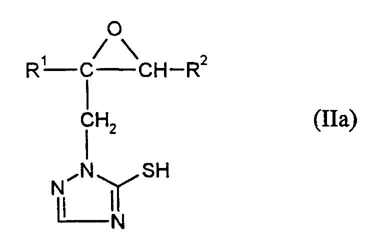 vorliegen. Es ist deshalb möglich, daß sich die erfindungsgemäßen Stoffe sowohl von der "Thiono"-Form der Formel (II) als auch von der "Mercapto"-Form der Formel (IIa) ableiten. Das bedeutet, daß die erfindungsgemäßen Stoffe entweder als Substanzen der Formel 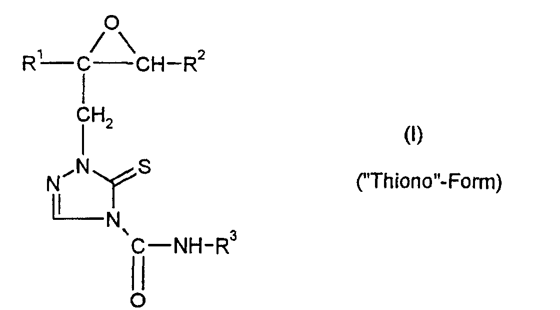 oder der Formel 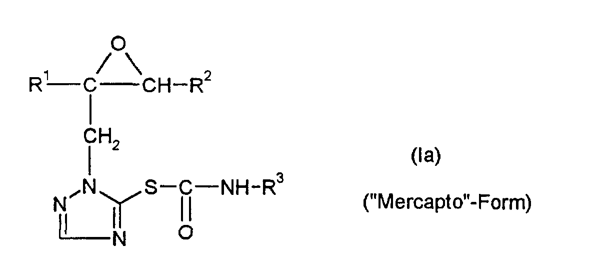 oder als Gemische der Formeln (I) und (Ia) vorliegen.

Als Beispiele für erfindungsgemäße Stoffe seien die in der folgenden Tabelle aufgeführten Oxiranyl-triazolinthione genannt.

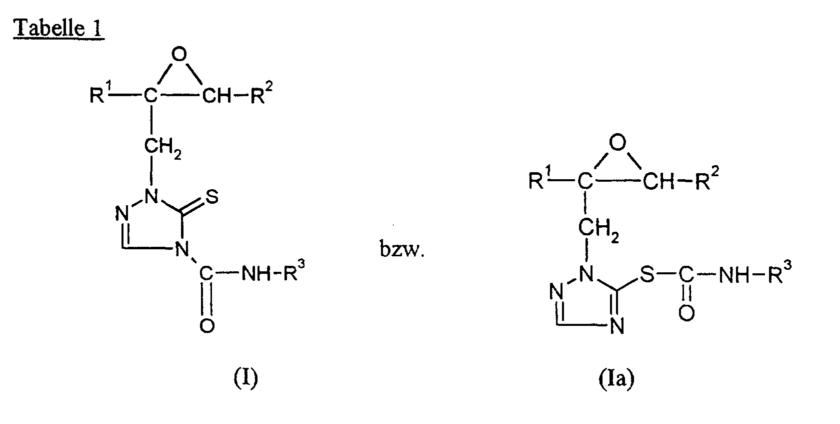

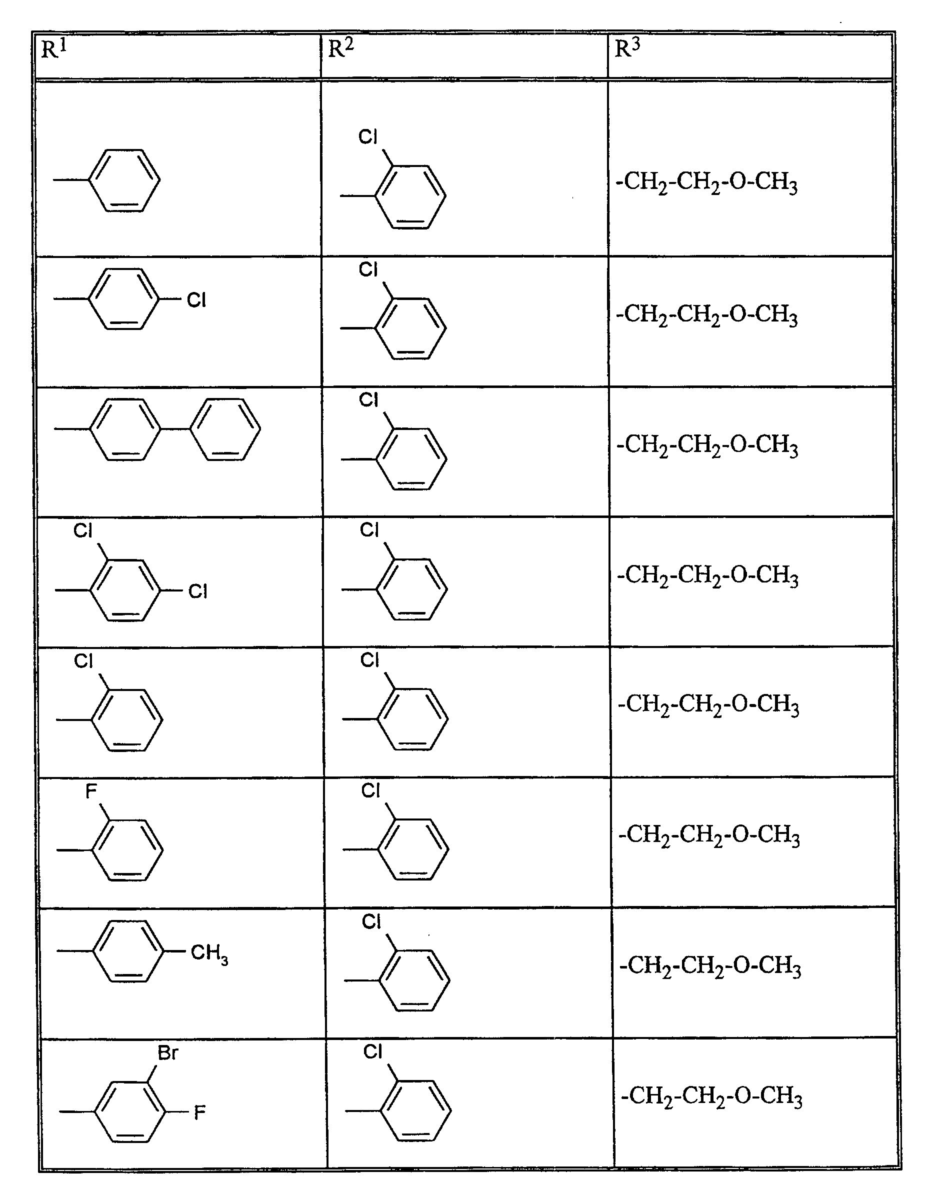

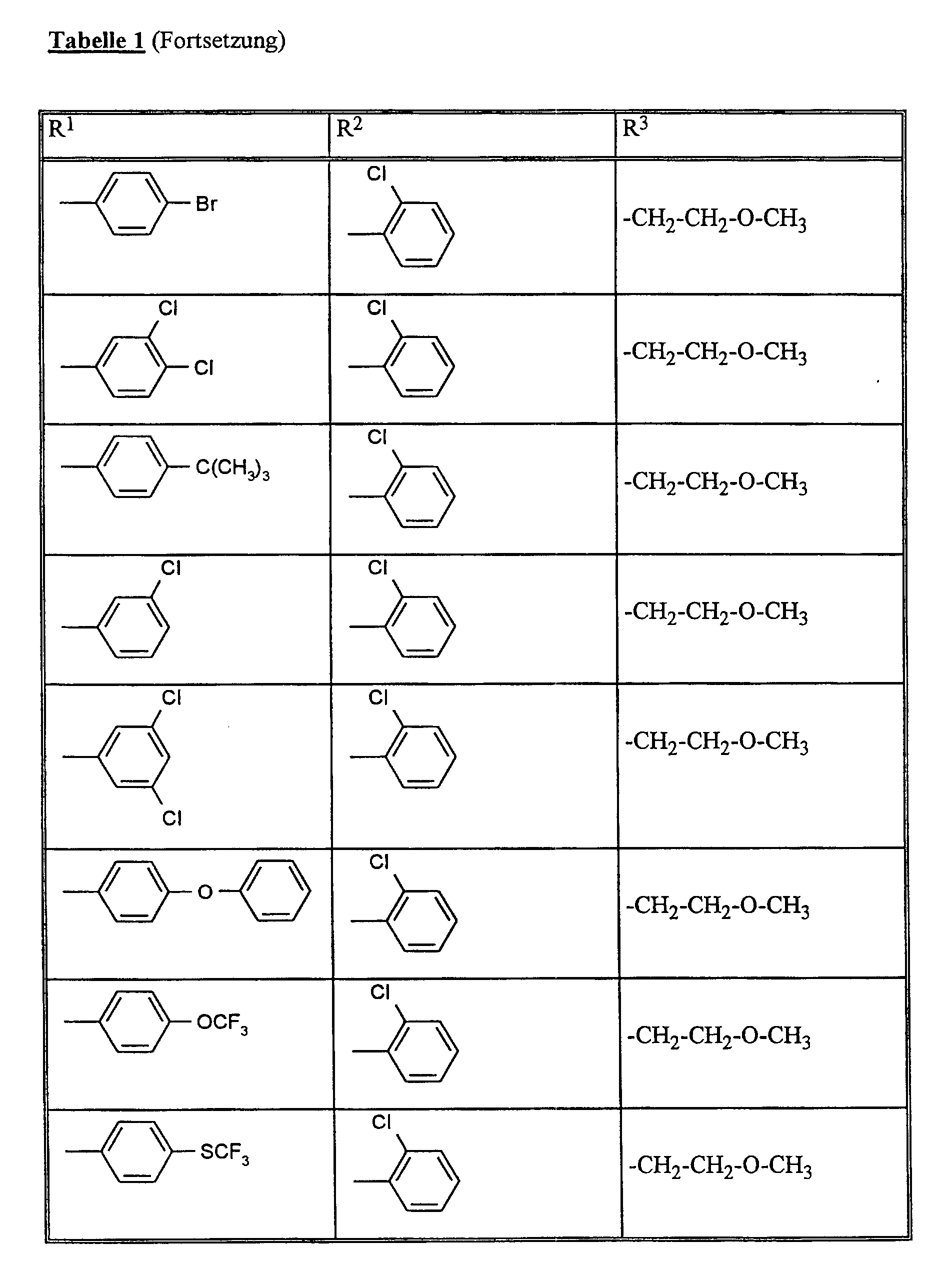

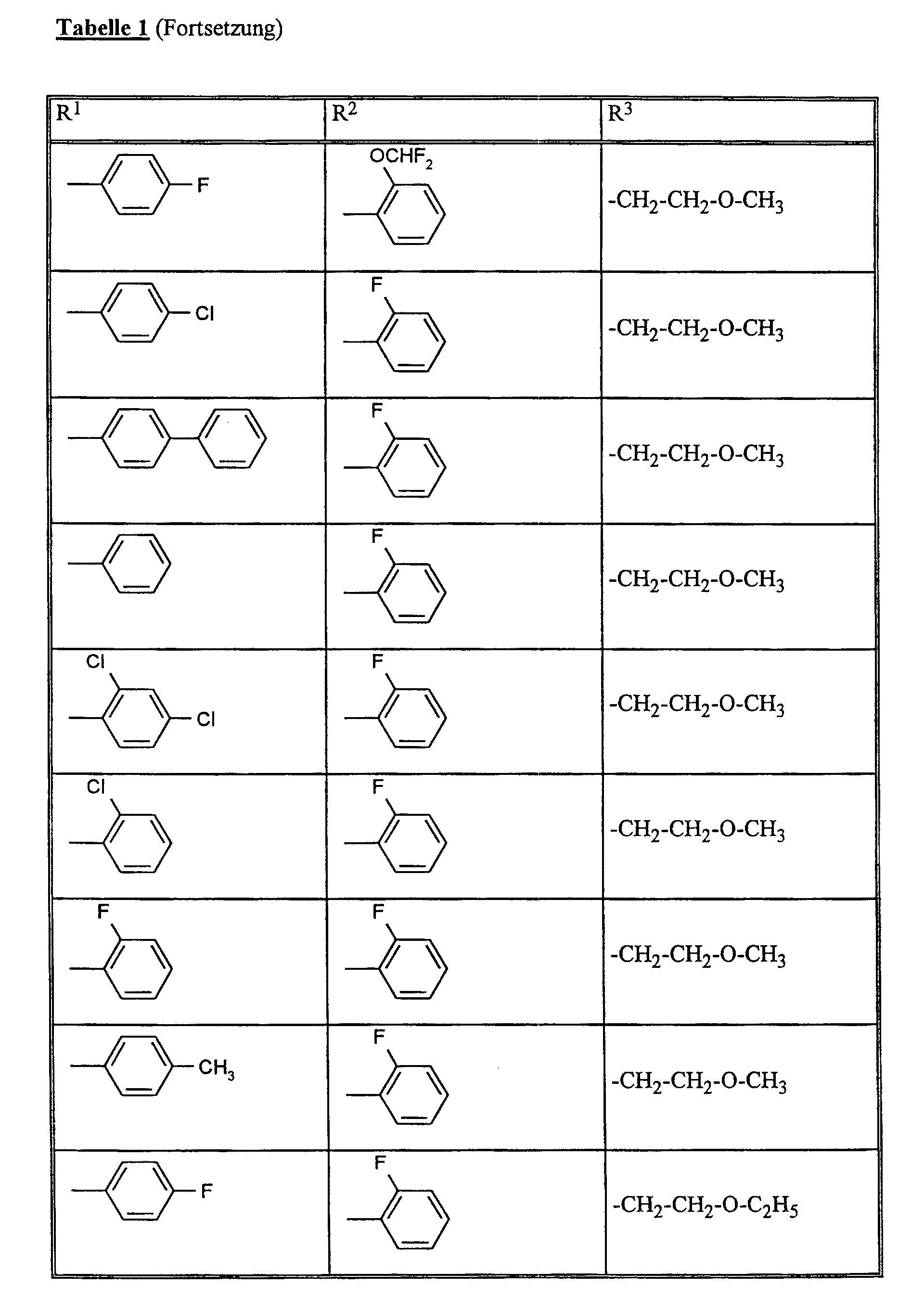

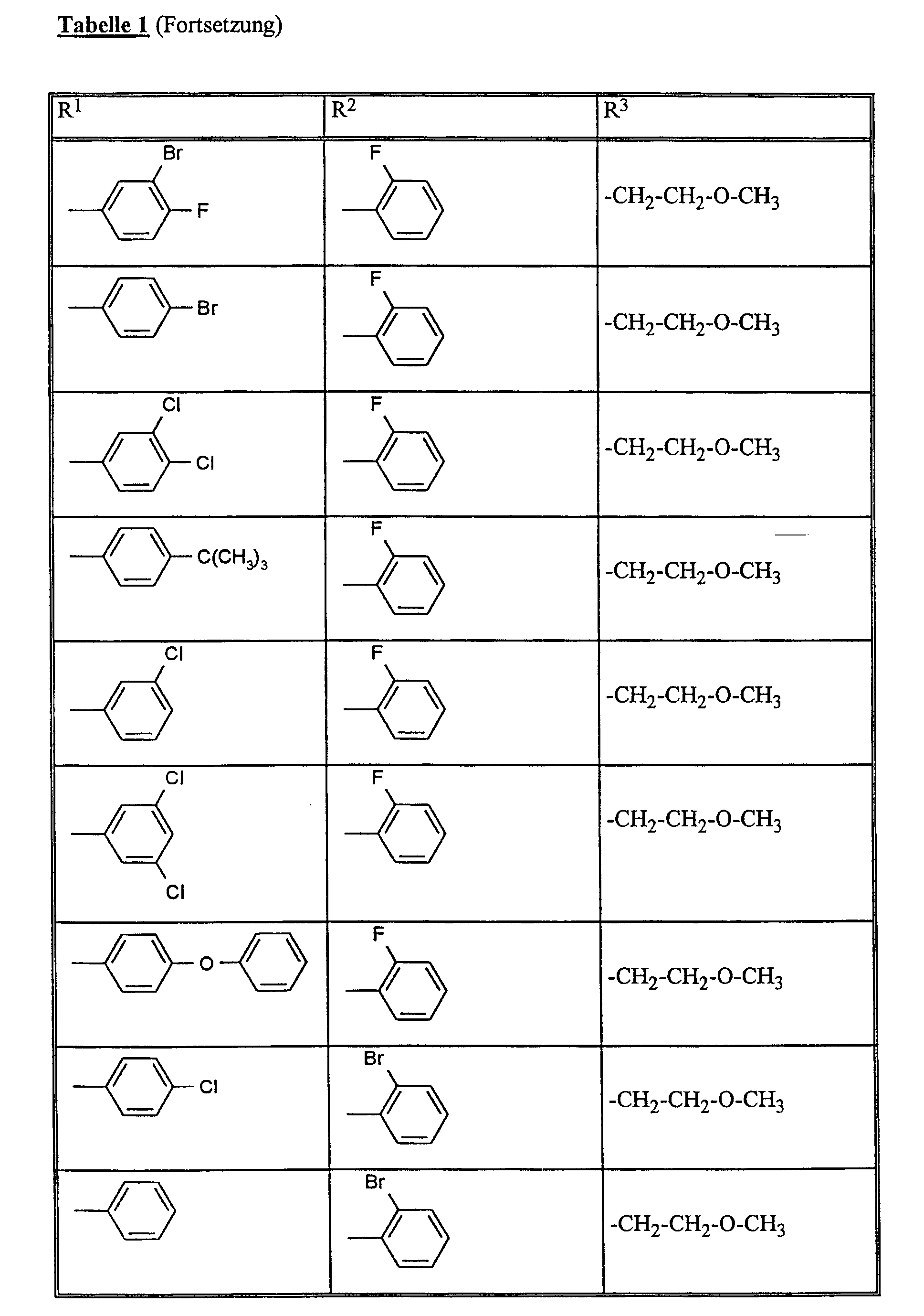

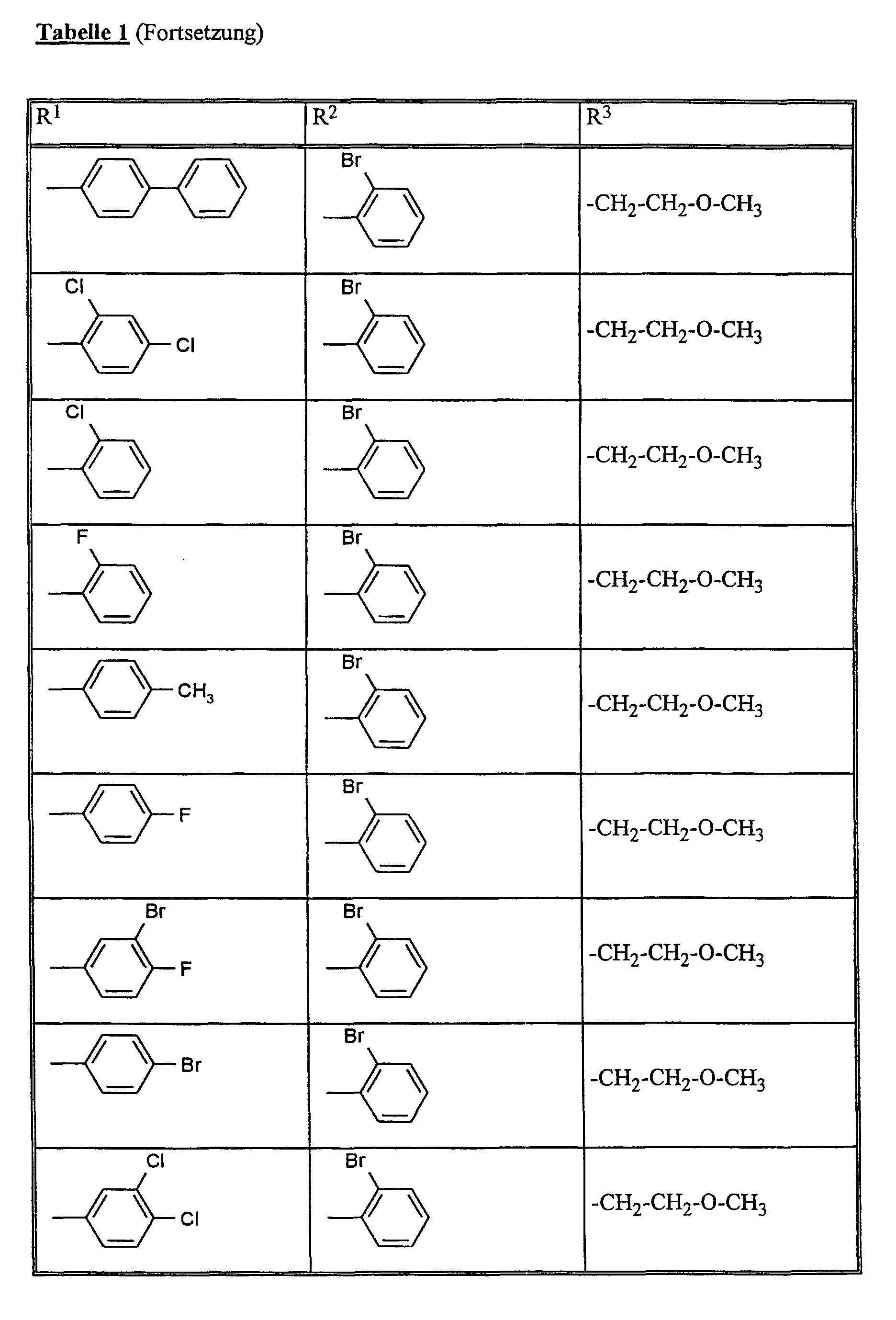

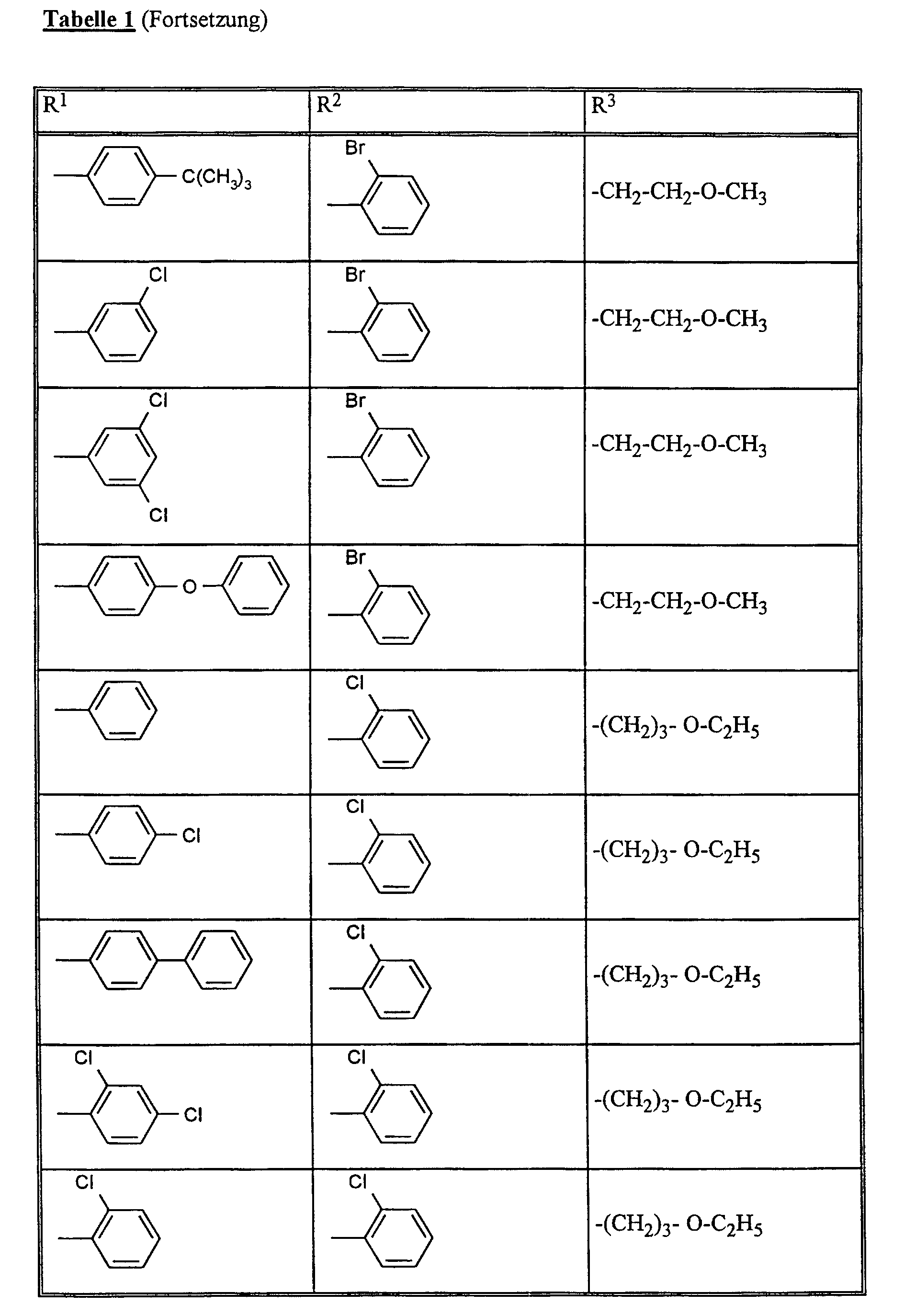

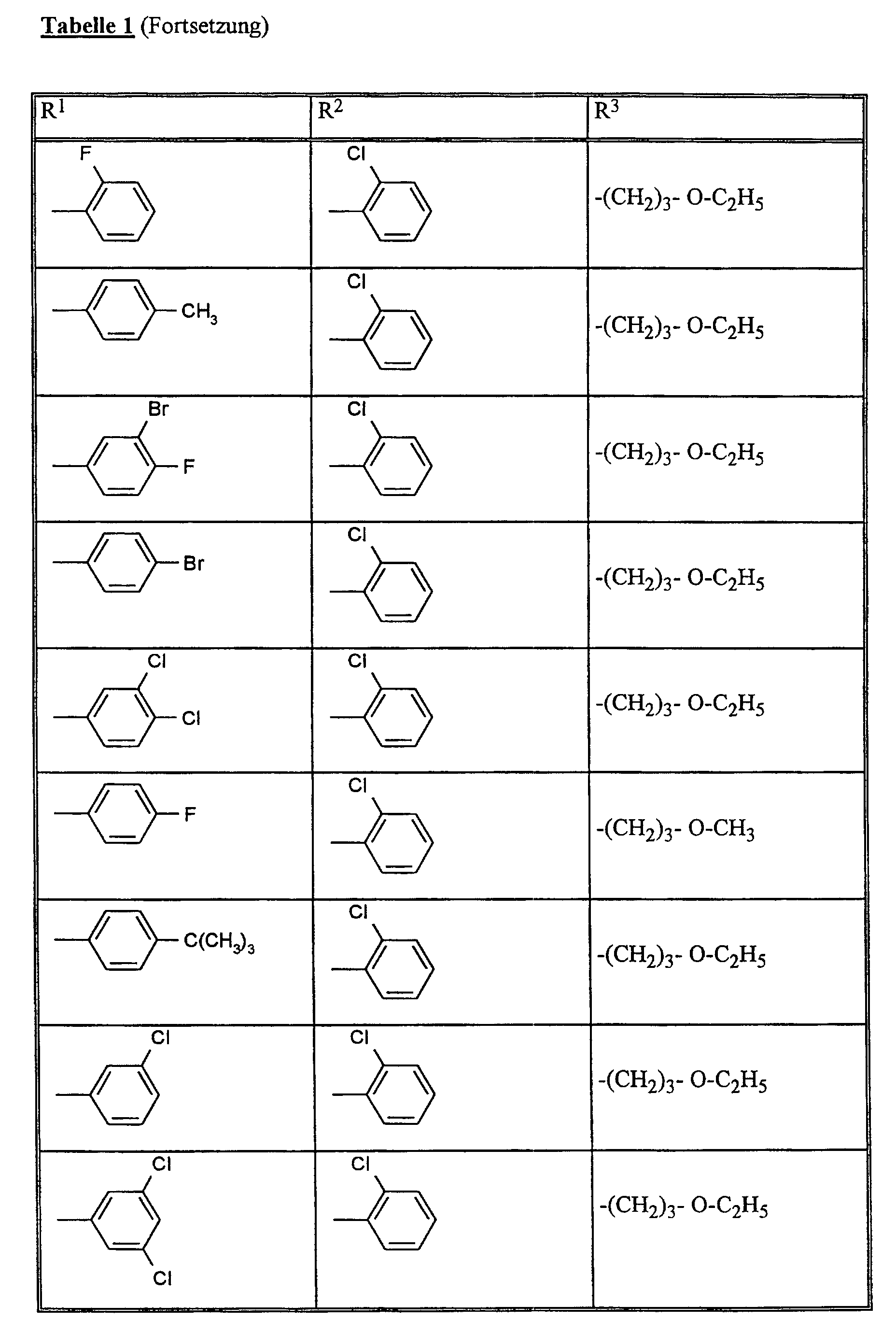

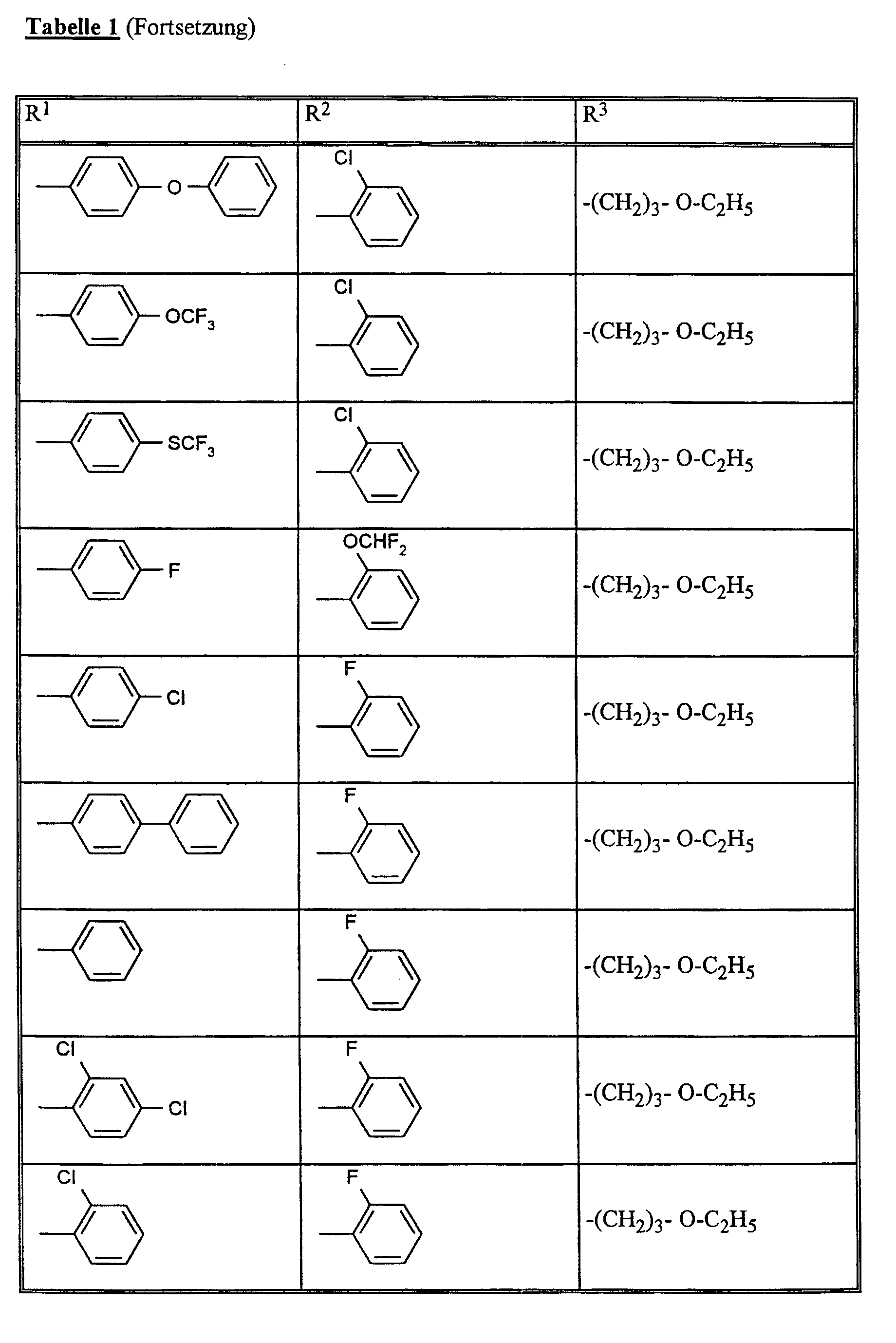

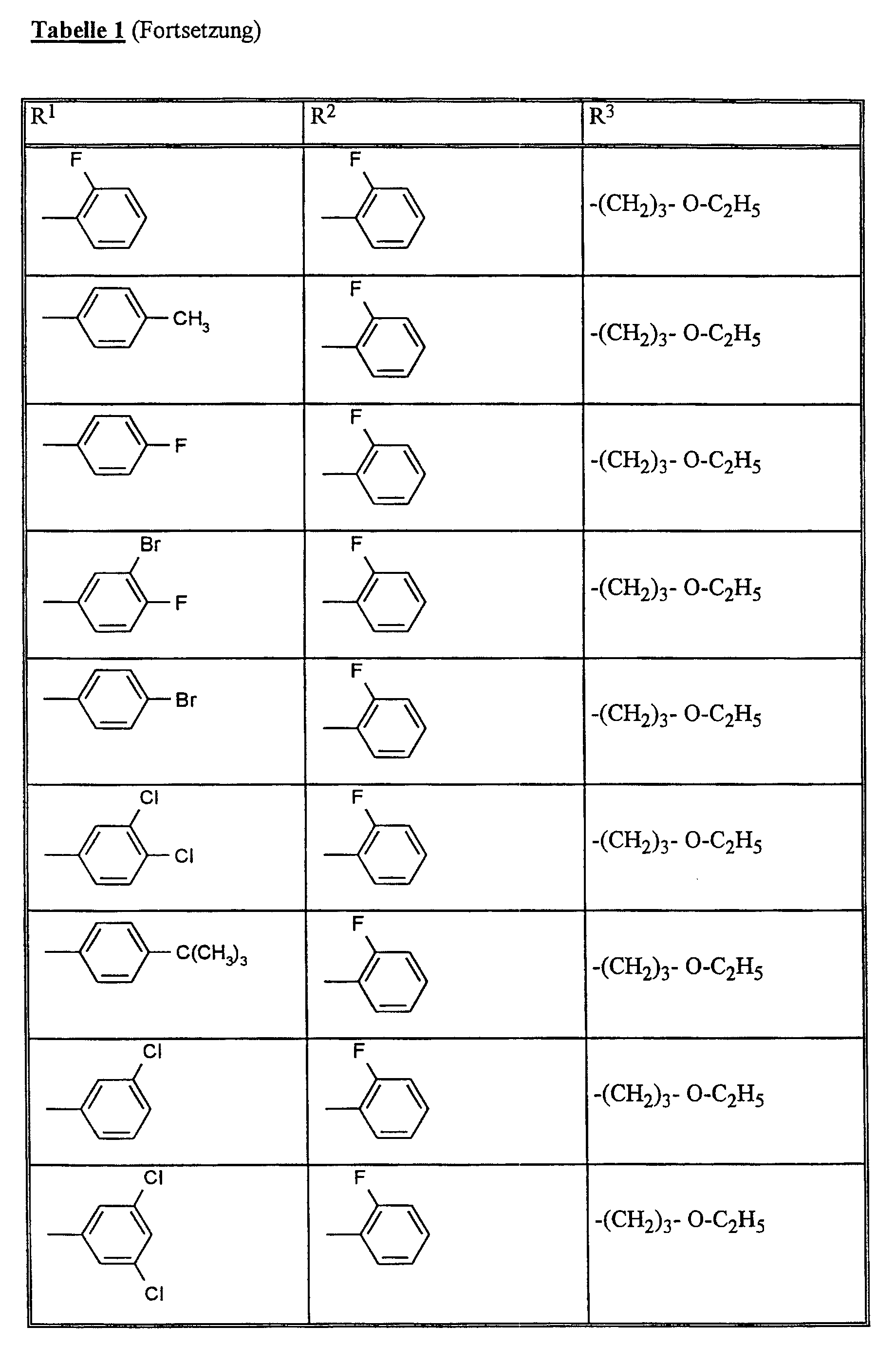

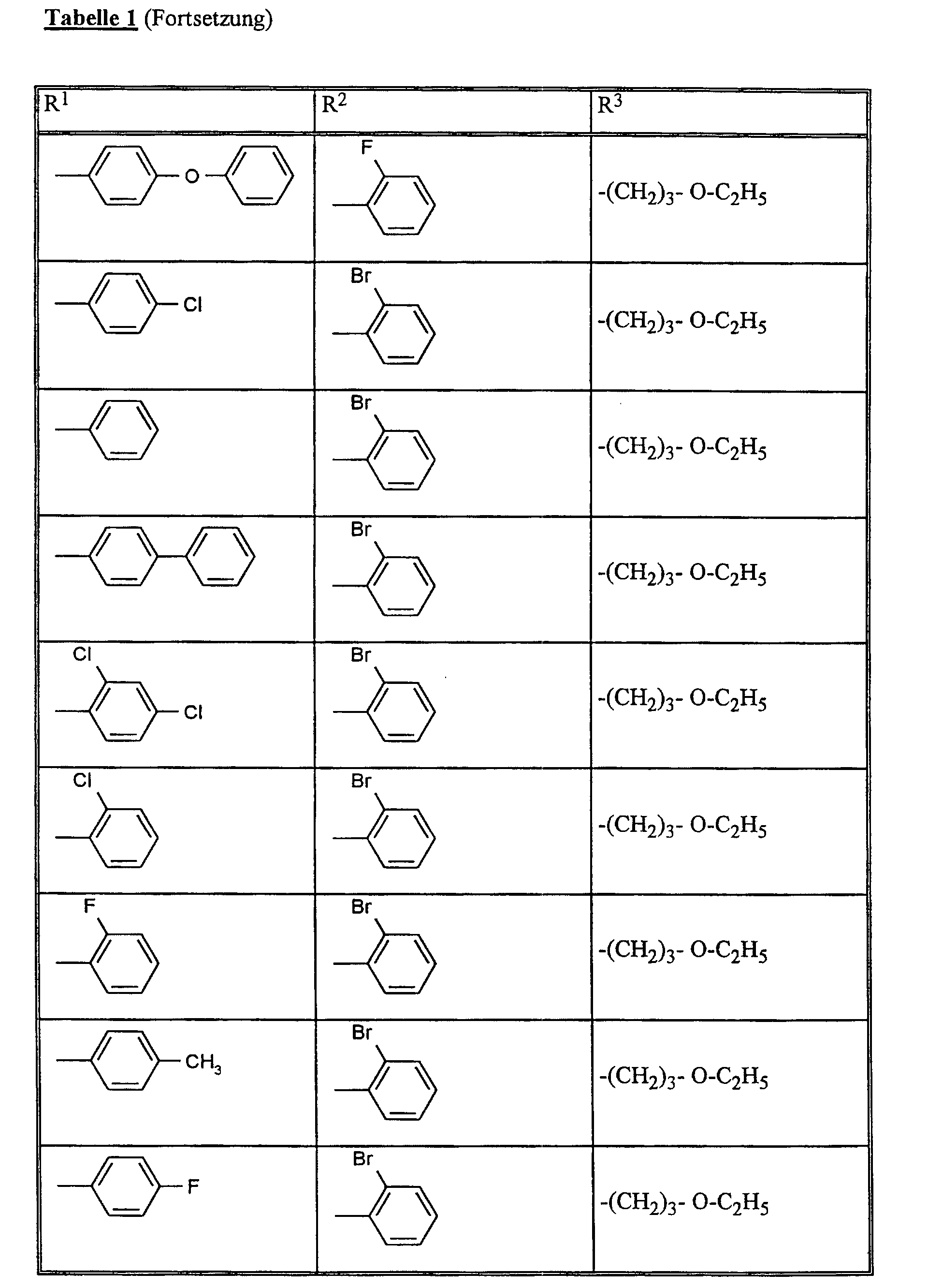

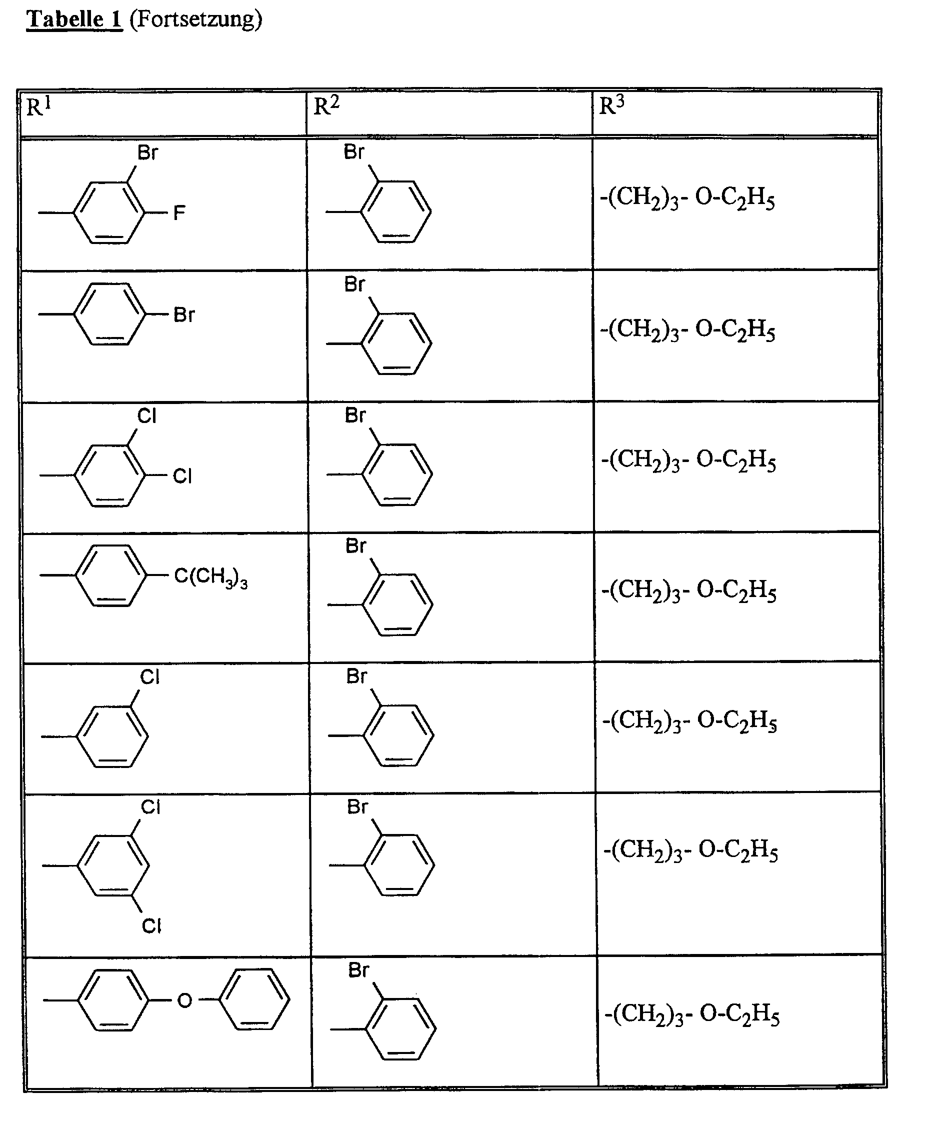

Verwendet man 3-(2-Chlor-phenyl)-2-(4-fluor-phenyl)-2-[(4,5-dihydro-5-thiono-1,2,4-triazol-1-yl)-methyl]-oxiran als Ausgangsstoff und 3-Ethoxy-propyl-isocyanat als Reaktionskomponente, so kann der Verlauf des erfindungsgemäßen Verfahrens durch das folgende Formelschema veranschaulicht werden.

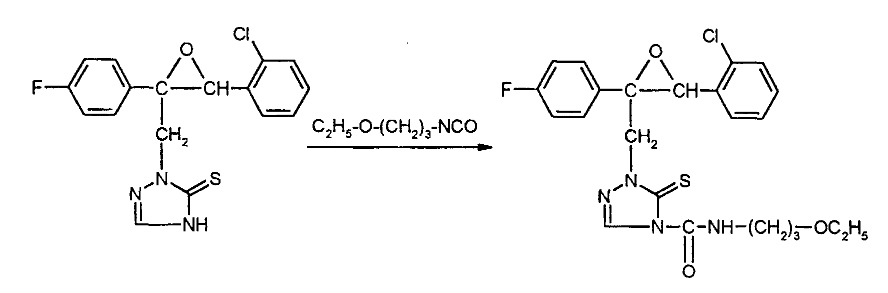

Die bei der Durchführung des erfindungsgemäßen Verfahrens als Ausgangsstoffe benötigten Oxiran-Derivate sind durch die Formel (II) bzw. (IIa) allgemein definiert. In dieser Formel haben R¹ und R² vorzugsweise diejenigen Bedeutungen, die bereits im Zusammenhang mit der Beschreibung der erfindungsgemäßen Stoffe der Formel (I) bzw. (Ia) vorzugsweise für diese Reste genannt wurden.

Die Oxiran-Derivate der Formel (II) bzw. (IIa) sind bereits bekannt (vgl. WO-A 96-38440).

Die bei der Durchführung des erfindungsgemäßen Verfahrens als Reaktionskomponenten benötigten Isocyanate sind durch die Formel (III) allgemein definiert. In dieser Formel hat R³ vorzugsweise diejenigen Bedeutungen, die bereits im Zusammenhang mit der Beschreibung der erfindungsgemäßen Stoffe der Formel (I) für diesen Rest genannt wurden.

Die Isocyanate der Formel (III) sind bekannt oder lassen sich nach bekannten Methoden herstellen.

Als Katalysatoren kommen bei der Durchführung des erfindungsgemäßen Verfahrens alle für derartige Umsetzungen üblichen Reaktionsbeschleuniger in Frage. Vorzugsweise verwendbar sind Amine, wie Triethylamin, Pyridin, Dimethylaminopyridin und Diazabicyclo-undecen (DBU).

Als Verdünnungsmittel kommen bei der Durchführung des erfindungsgemäßen Verfahrens alle für derartige Umsetzungen üblichen, inerten organischen Solventien in Frage. Vorzugsweise verwendbar sind aromatische Kohlenwasserstoffe, wie Toluol, Xylol oder Decalin, ferner halogenierte Kohlenwasserstoffe, wie Dichlormethan, Chloroform, Tetrachlormethan, Dichlorethan oder Trichlorethan, außerdem Ether, wie Diethylether, Diisopropylether, Methyl-tert.-butylether, Methyl-tert.-amylether, Dioxan oder Tetrahydrofuran, und weiterhin Nitrile, wie Acetonitril, Propionitril, n- oder iso-Butyronitril.

Die Reaktionstemperaturen können bei der Durchführung des erfindungsgemäßen Verfahrens innerhalb eines bestimmten Bereiches variiert werden. Im allgemeinen arbeitet man bei Temperaturen zwischen -20°C und +100°C, vorzugsweise zwischen 0°C und +80°C.

Bei der Durchführung des erfindungsgemäßen Verfahrens arbeitet man im allgemeinen unter Atmosphärendruck. Es ist aber auch möglich, unter erhöhtem Druck oder, sofern keine leicht flüchtigen Komponenten an der Umsetzung beteiligt sind, auch unter vermindertem Druck zu arbeiten.

Bei der Durchführung des erfindungsgemäßen Verfahrens setzt man auf 1 mol an Oxiran-Derivat der Formel (II) bzw. (IIa) im allgemeinen 1 bis 1,5 mol an Isocyanat der Formel (III) sowie eine geringe Menge an Katalysator ein. Die Aufarbeitung erfolgt nach üblichen Methoden. Im allgemeinen geht man so vor, daß man das Reaktionsgemisch unter vermindertem Druck einengt und das verbleibende Produkt nach üblichen Methoden, z.B. durch Umkristallisation oder Chromatographie, von noch vorhandenen Verunreinigungen befreit.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich insbesondere zur Bekämpfung von Pyricularia oryzae an Reis sowie zur Bekämpfung von Getreidekrankheiten, wie Puccinia-, Erysiphe- und Fusarium-Arten. Außerdem lassen sich die erfindungsgemäßen Stoffe sehr gut gegen Venturia, Podosphaera und Sphaerotheca einsetzen. Sie besitzen darüber hinaus auch eine sehr gute in-vitro Wirkung

Die erfindungsgemäßen Wirkstoffe eignen sich auch zur Steigerung des Ernteertrages. Sie sind außerdem mindertoxisch und weisen eine gute Pflanzenverträglichkeit auf.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
   Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox, Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
   Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Pimaricin, Piperalin, Polyoxin, Polyoxorim, Probenazole, Prochloraz, Procymidon, Propamocarb, Propanosine-Natrium, Propiconazol, Propineb, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,
   α-(1,1-Dimethylethyl)-β-(2-phenoxyethyl)-1H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-a-(Methoxyimino)-N-methyl-2-phenoxy-phenylacetamid,
   {2-Methyl-1-[[[1-(4-methylphenyl)-ethyl]-amino]-carbonyl]-propyl}-carbaminsäure-1-isopropylester
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3 -[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-methanamin, 8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat, cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholinhydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin-Natriumsalz,
   O,O-Diethyl-[2-(dipropylamino)-2-oxoethyl]-ethylphosphoramidothioat,
   O-Methyl-S-phenyl-phenylpropylphosphoramidothioat,
   S-Methyl-1,2,3-benzothiadiazol-7-carbothioat,
   spiro[2H]-1-Benzopyran-2,1'(3'H)-isobenzofuran]-3'-on,
**Bakterizide:**
   Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
   Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lambda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, Nitenpyram,
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
   Quinalphos,
   Salithion, Sebufos, Silafluofen, Sulfotep, Sulprofos,
   Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die zum Schutz technischer Materialien verwendeten Mittel enthalten die Wirkstoffe im allgemeinen in einer Menge von 1 bis 95 Gewichts-%, bevorzugt von 10 bis 75 Gewichts-%.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung von erfindungsgemäßen Wirkstoffen werden durch die folgenden Beispiele veranschaulicht.

### Herstellungsbeispiele

### Beispiel 1

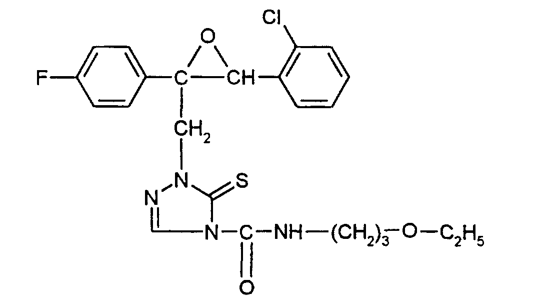

In ein Gemisch aus 350 mg (0,97 mmol) 3-(2-Chlor-phenyl)-2-(4-fluor-phenyl)-2-[(4,5-dihydro-5-thiono-1,2,4-triazol-1-yl)-methyl]-oxiran, 0,1 ml Triethylamin und 5 ml absolutem Tetrahydrofuran wird bei Raumtemperatur unter Rühren eine Lösung von 110 mg (0,97 mmol) 3-Ethoxy-propyl-isocyanat in 5 ml absolutem Tetrahydrofuran eingetropft. Nach beendeter Zugabe wird das Reaktionsgemisch 1 Stunde auf 60°C erhitzt und dann unter vermindertem Druck eingeengt. Das verbleibende Produkt wird mit einem Gemisch von Cyclohexan Ethylacetat = 4:1 an Kieselgel chromatographiert. Nach dem Einengen des Eluates erhält man 390 mg (82 % der Theorie) an der Substanz der oben angegebenen Formel.

¹H-NMR-Spektrum (300 MHz, CDCl₃, TMS):
- δ =: 10,0 (s, 1H); 8,4 (s, 1H); 7,6-7,3 (m, 6H); 7,0 (t, 2H, J=8,7 Hz); 5,1 (d, 1H, J=14,9 Hz); 4,1 (s, 1H); 3,7 (d, 1H, J=14,9 Hz); 3,5-3,4 (m, 6H); 1,9-1,8 (m, 2H); 1,2 (t, 3H, J= 7,0 Hz) ppm.

Nach der im Beispiel 1 angegebenen Methode werden auch die in der folgenden Tabelle aufgeführten Verbindungen hergestellt:

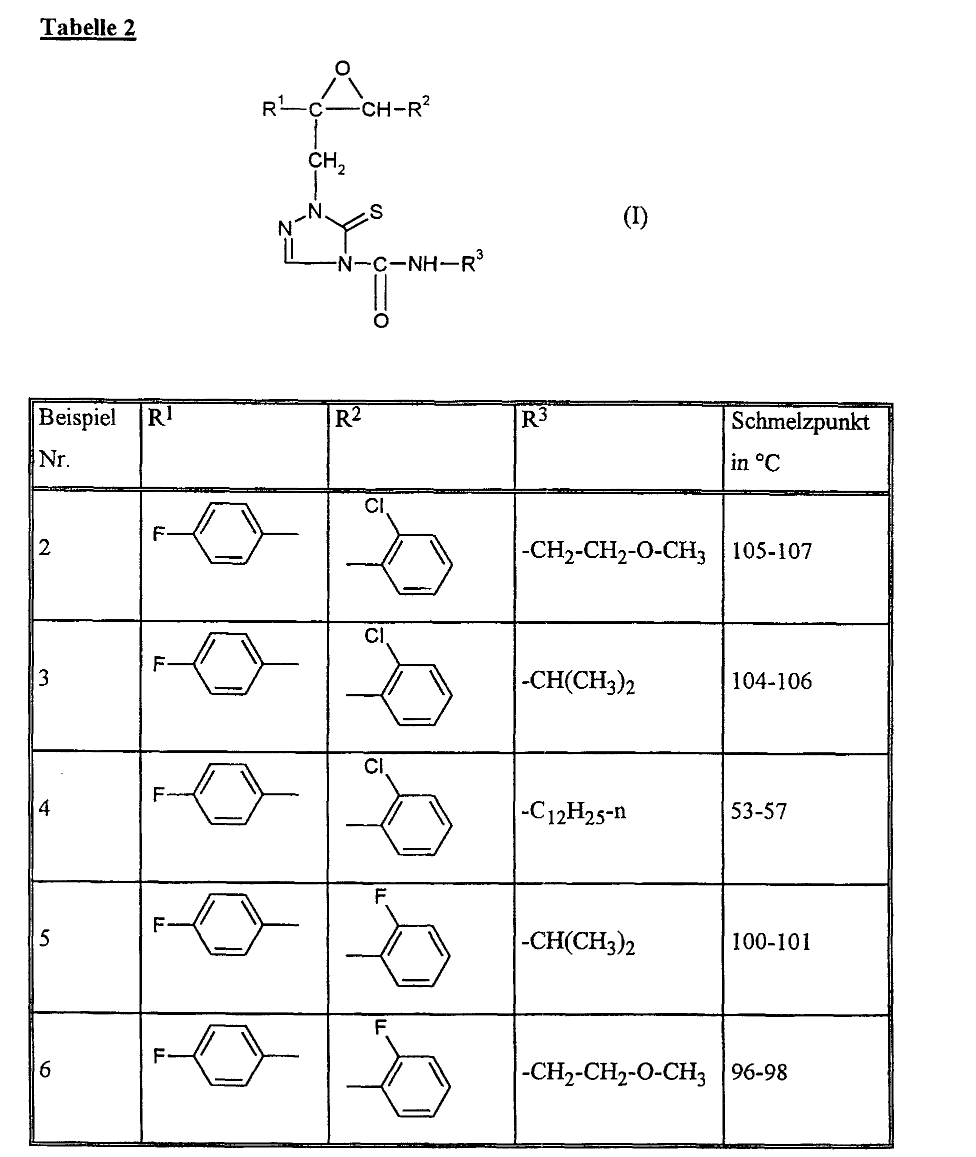

### Beispiel 7

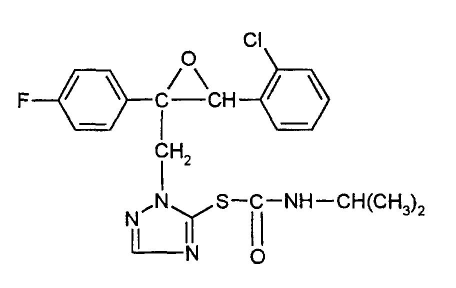

¹H-NMR-Spektrum (400 Mhz, CDCl₃, TMS):
δ= 7,8 (s, 1H); 7,3-7,1 (m, 4H); 7,0-6,9 (m, 4H); 6,7 (s, 1H); 5,0 (d, 1H); 4,7 (d, 1H); 4,5 (s, 1H); 3,7 (m, 1H); 1,0 (d, 6H, J= 6,6 Hz) ppm.

### Verwendungsbeispiele

### Beispiel A

### Erysiphe-Test (Gerste) / protektiv

- Lösungsmittel:: 25 Gew.-Teile N,N-Dimethylacetamid
- Emulgator:: 0,6 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

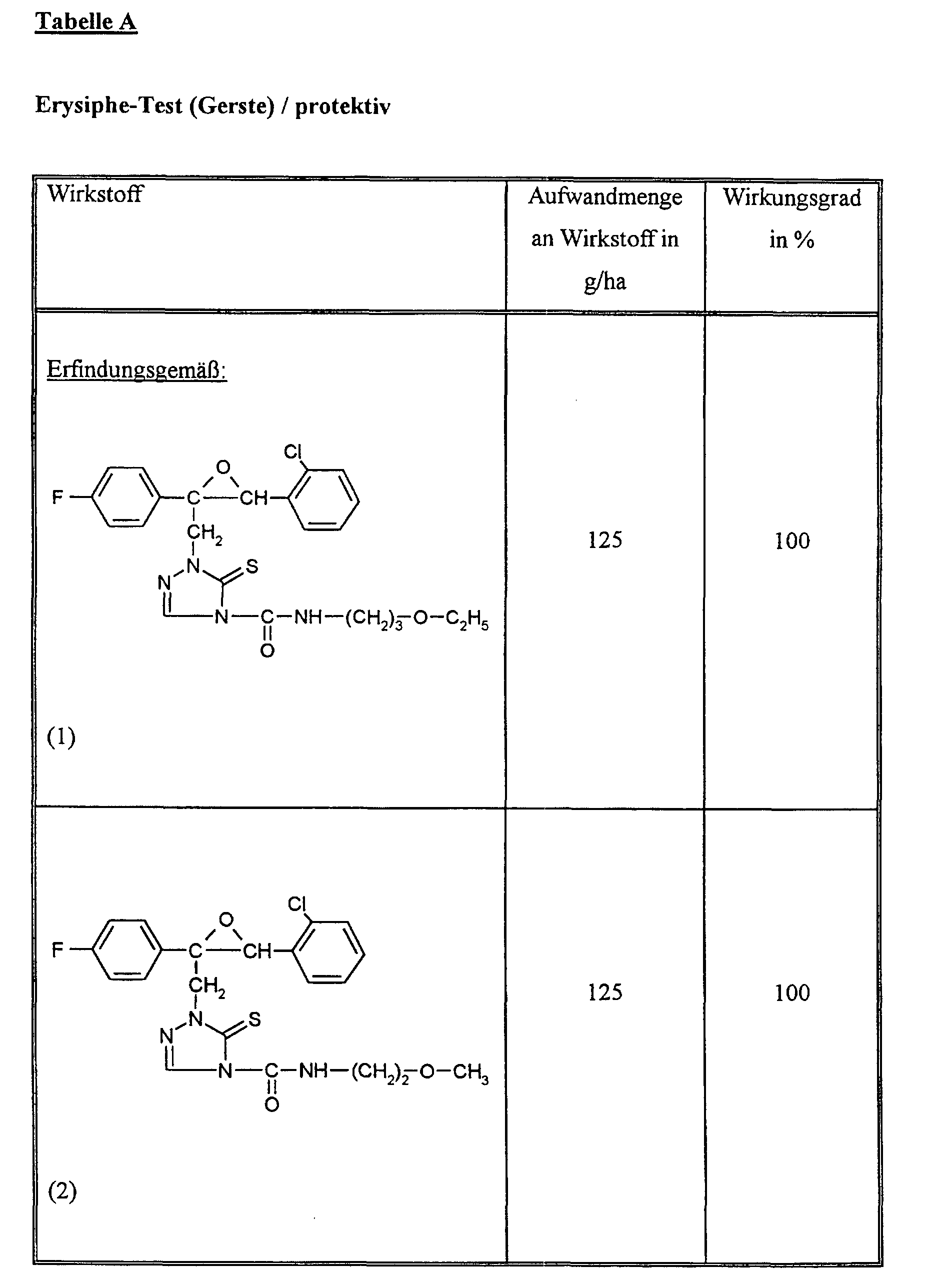

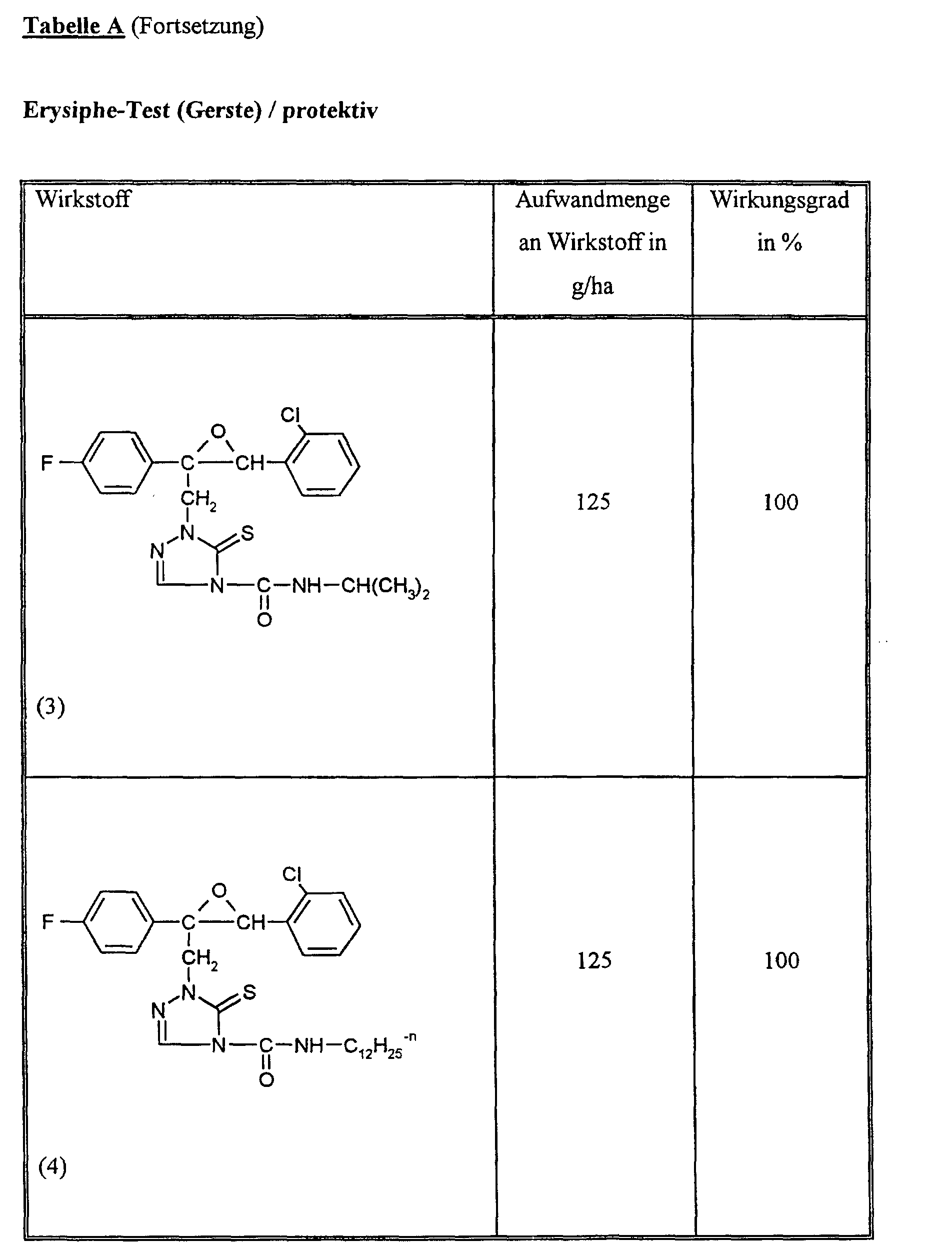

### Beispiel B

### Erysiphe-Test (Gerste) / kurativ

- Lösungsmittel:: 25 Gew.-Teile N,N-Dimethylacetamid
- Emulgator:: 0,6 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

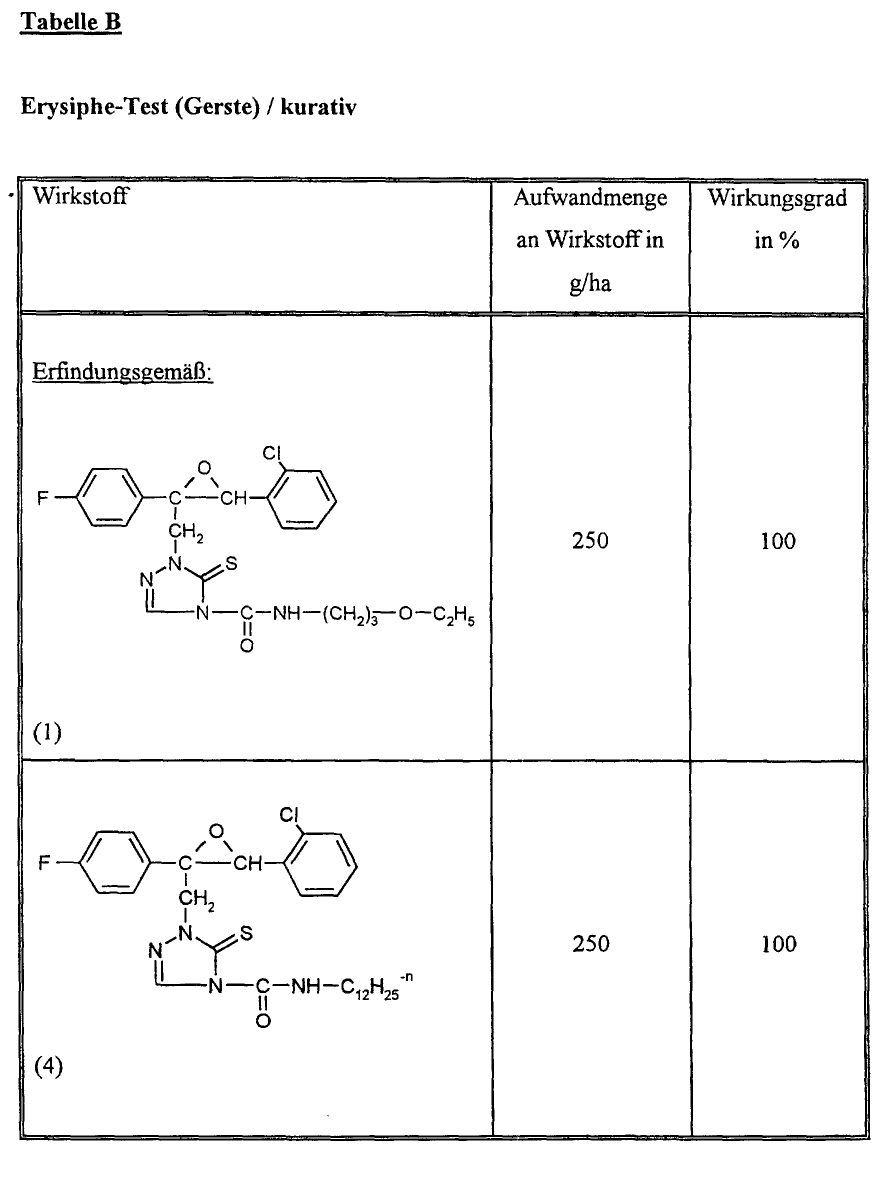

### Beispiel C

### Erysiphe-Test (Weizen) / protektiv

- Lösungsmittel:: 25 Gew.-Teile N,N-Dimethylacetamid
- Emulgator:: 0,6 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

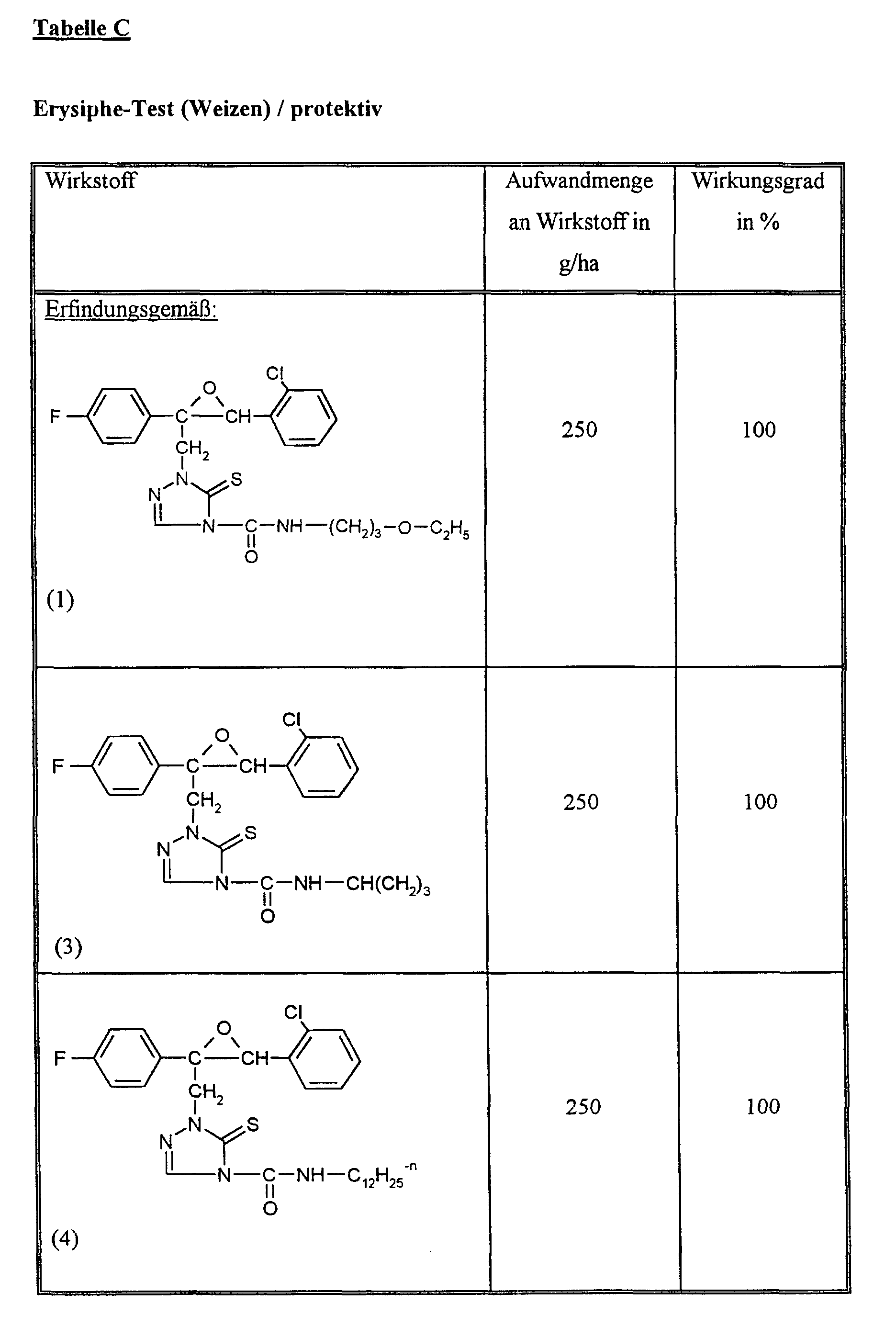

### Beispiel D

### Leptosphaeria nodorum-Test (Weizen) / protektiv

- Lösungsmittel:: 25 Gewichtsteile N,N-Dimethylacetamid
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von 80 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

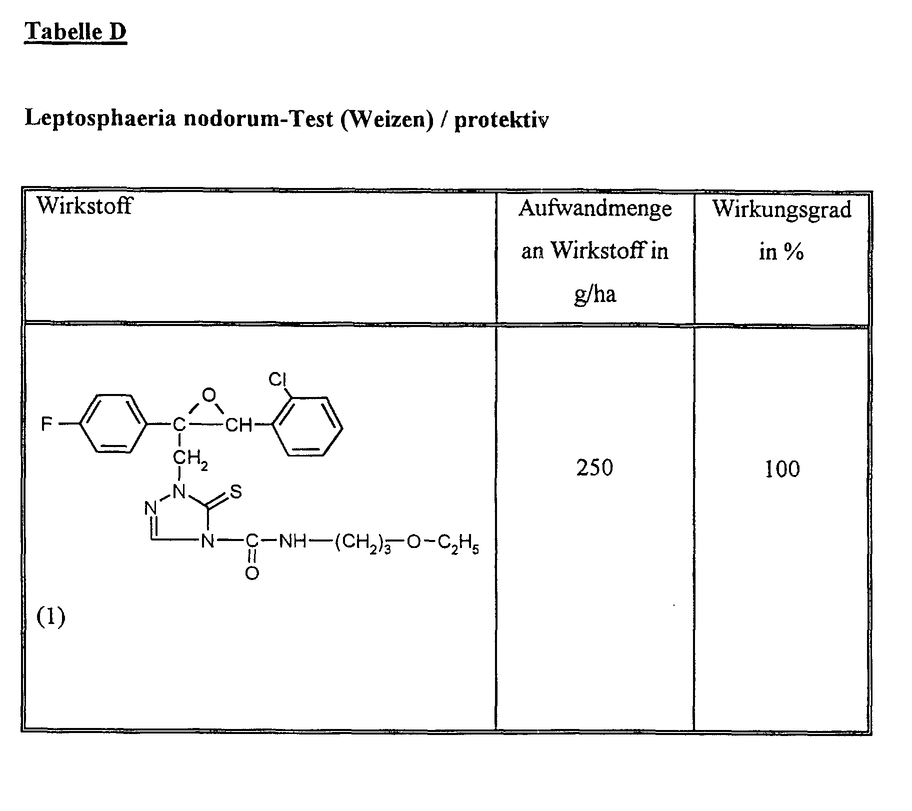

### Beispiel E

### Puccinia-Test (Weizen) / kurativ

- Lösungsmittel:: 25 Gewichtsteile N,N-Dimethylacetamid
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Kondiensuspension von Puccinia recondita besprüht. Die Pflanzen verbleiben 48 Stunden bei 20°C und 100 % relativer Luftfeuchte in einer Inkubationskabine. Anschließend werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

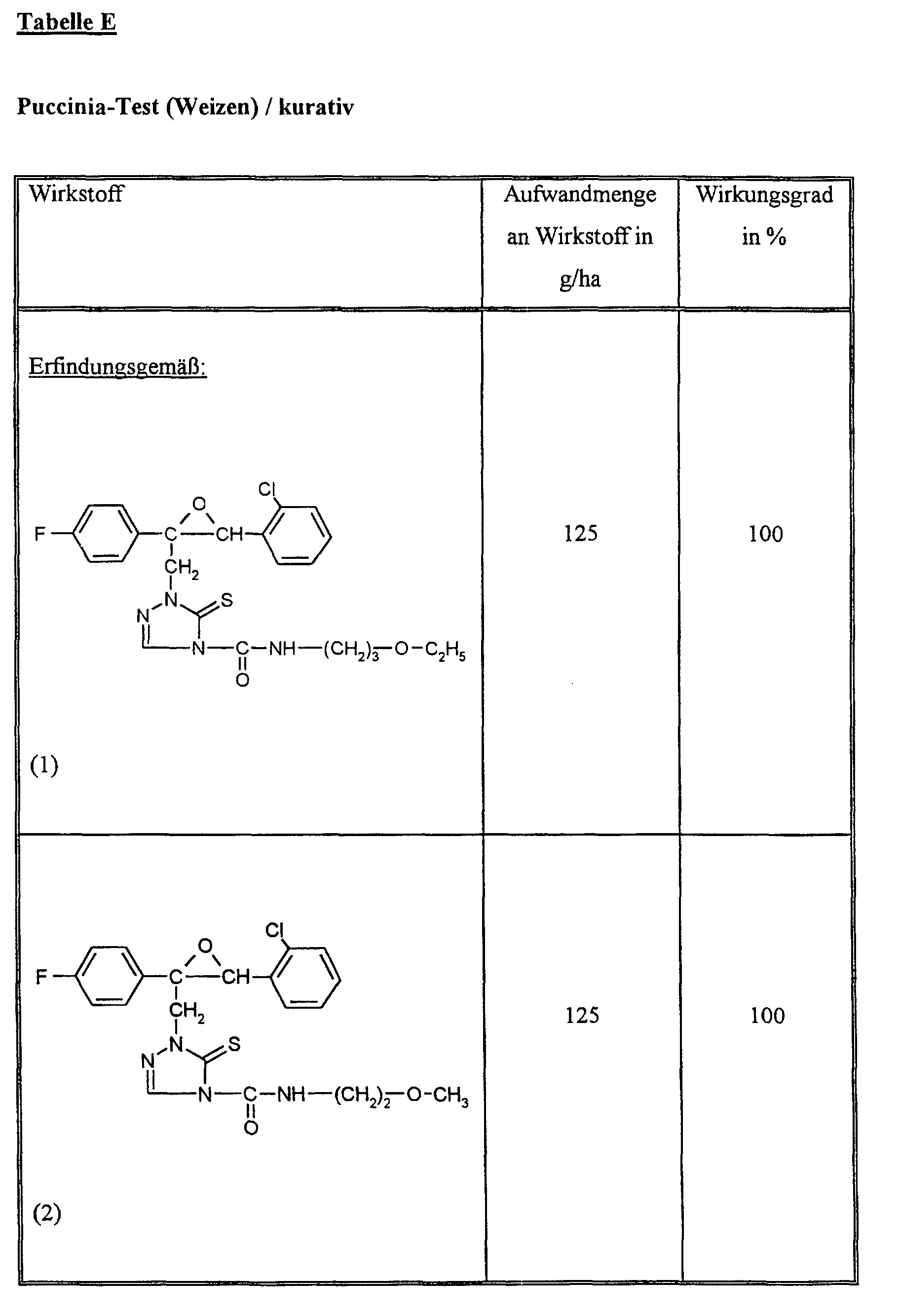

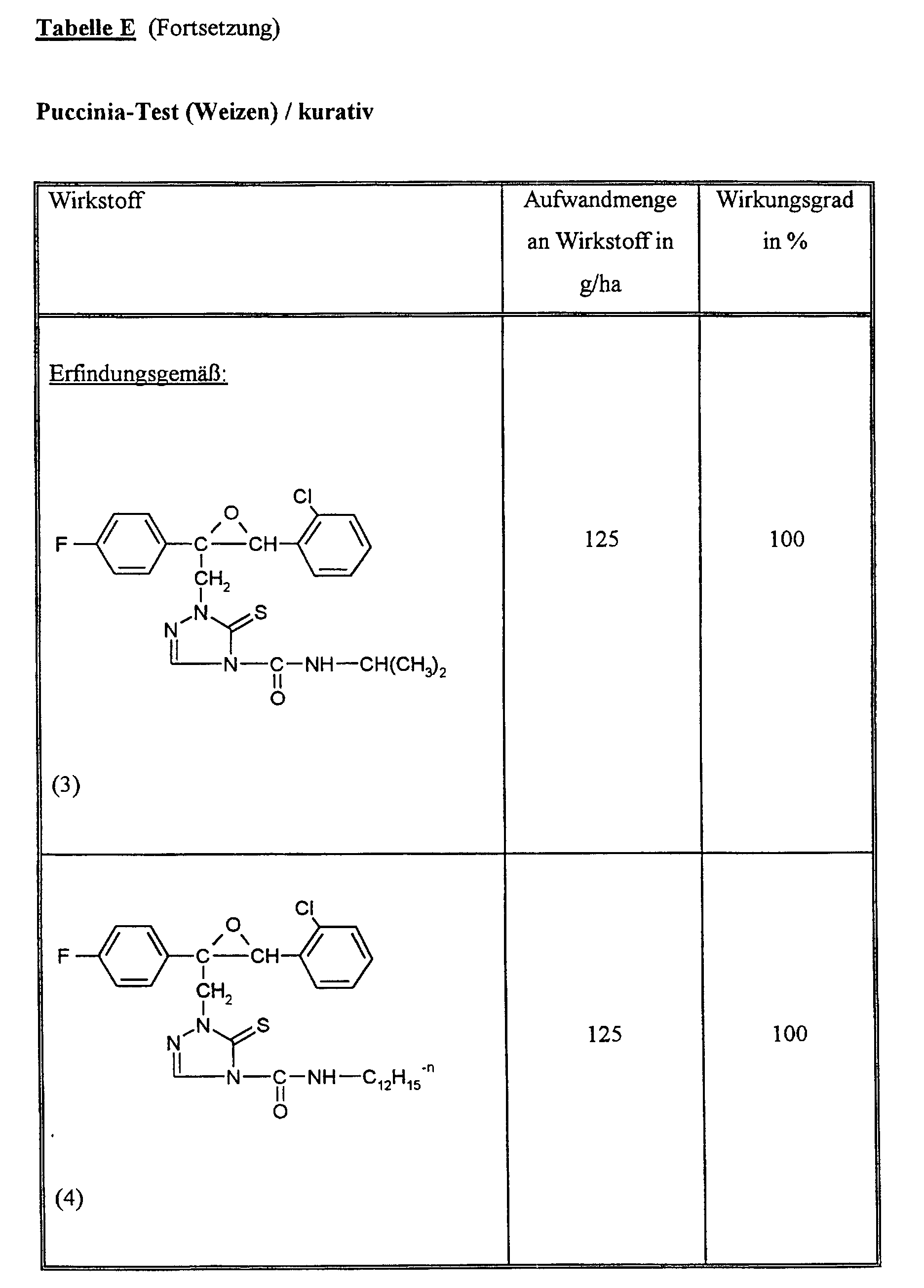

### Beispiel F

### Puccinia-Test (Weizen) / protektiv

- Lösungsmittel:: 25 Gewichtsteile N,N-Dimethylacetamid
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

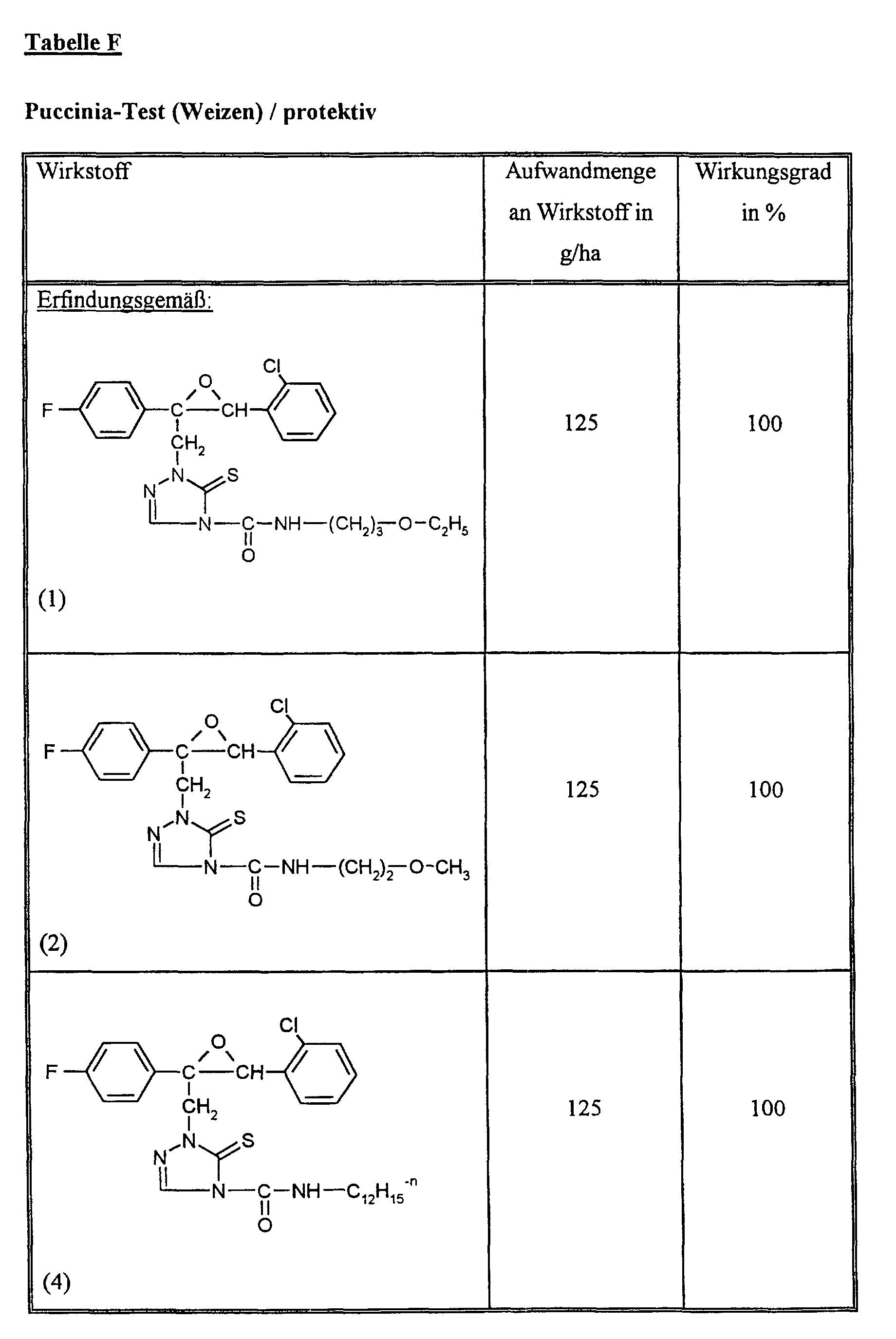

### Beispiel G

### Podosphaera-Test (Apfel) / protektiv

- Lösungsmittel:: 47 Gewichtsteile Aceton
- Emulgator:: 3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension des Apfelmehltauerregers ***Podosphaera leucotricha*** inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

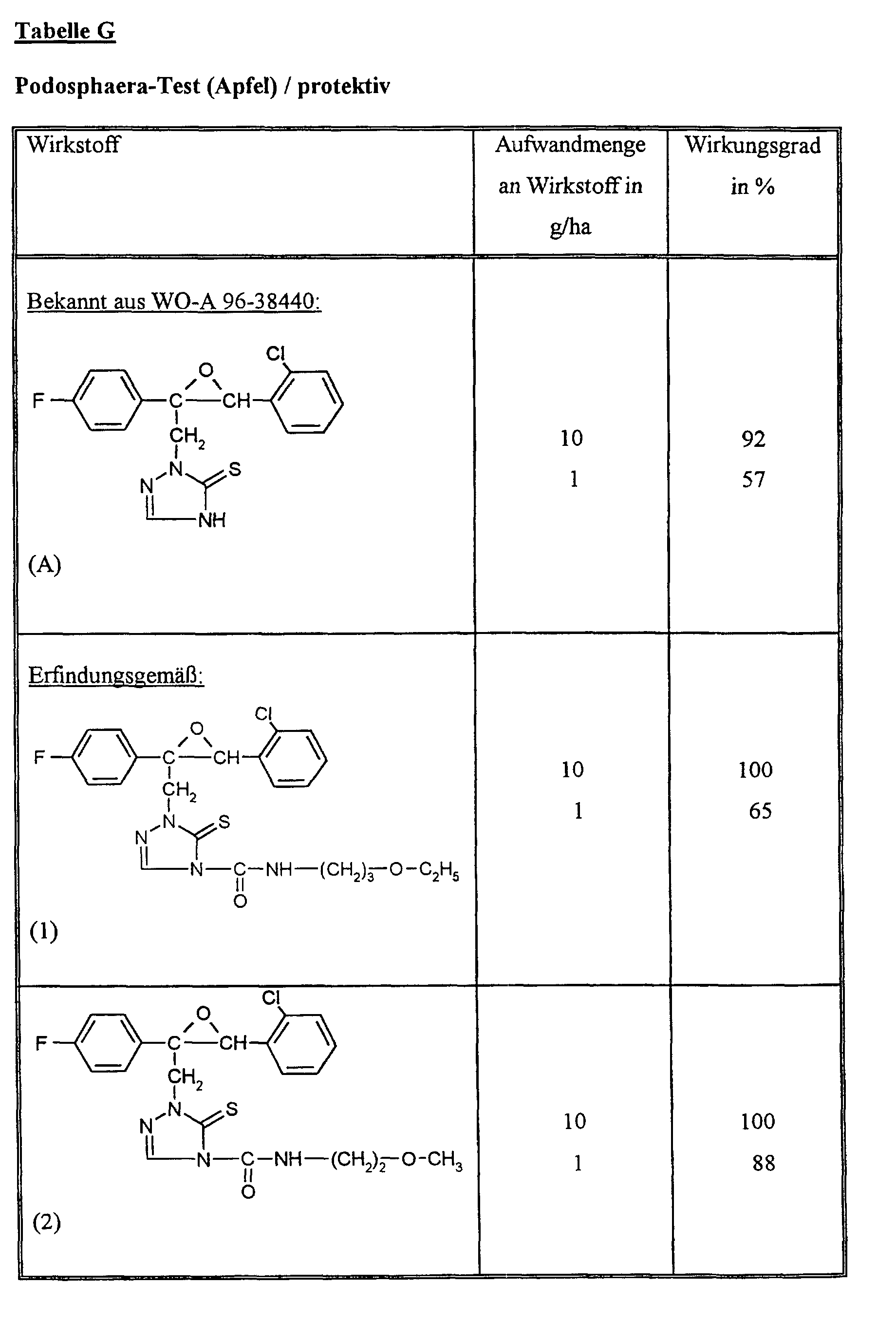

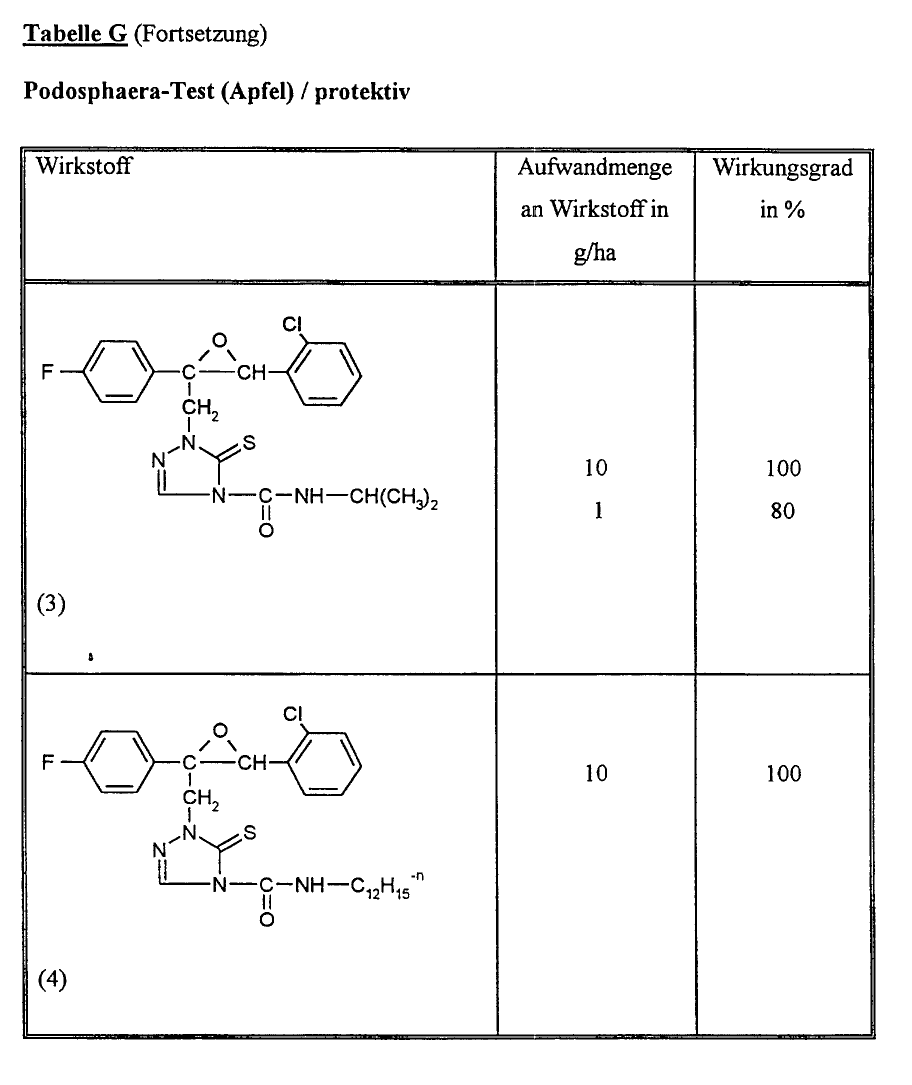

### Beispiel H

### Hemmtest an Riesenkolonien von Basidiomyceten

- Lösungsmittel:: Dimethylsulfoxid

Zur Herstellung einer zweckmäßigen Wirkstoffzubereitung werden 0,2 Gewichtsteile Wirkstoff mit 99,8 Gewichtsteilen des oben angegebenen Lösungsmittels versetzt.

Ein unter Verwendung von Malzextrakt-Pepton hergestellter Agar wird in flüssigem Zustand mit der Wirkstoffzubereitung in der jeweils gewünschten Aufwandmenge vermischt. Nach dem Aushärten wird der so hergestellte Nährstoffboden bei 26°C mit Mycelstücken inkubiert, die aus Kolonien von Coriolus versicolor ausgestochen wurden.

Nach 3- bzw. 7-tägiger Lagerung bei 26°C erfolgt die Auswertung, indem das Hyphenwachstum gemessen und die im Vergleich zur unbehandelten Kontrolle aufgetretene Hemmung in Prozent bonitiert wird. Dabei bedeutet 0% eine Wuchshemmung, die derjenigen der unbehandelten Kontrolle entspricht, während eine Wuchshemmung von 100 % bedeutet, daß kein Hyphenwachstum beobachtet wird.

Wirkstoffe, Wirkstoffkonzentrationen und Versuchsergebnisse gehen aus der folgenden Tabelle hervor.

**Tabelle H**

| Hemmtest an Riesenkolonien von Basidiomyceten | | | |
|---|---|---|---|
| Pilzspecies | Prozentuale Hemmung des radialen Wachstums von Riesenkolonien bei 6 ppm an Wirkstoff gemäß Beispiel | | |
| | (1) | (3) | (5) |
| Coriolus versicolor | 100 | 100 | 100 |

## Patentansprüche

1. Oxiranyl-triazolinthione der Formel 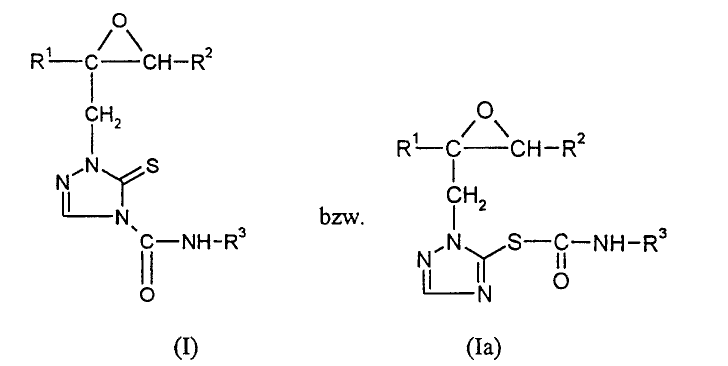 worin
R¹ für Alkyl mit 1 bis 4 Kohlenstoffatomen, Halogenalkyl mit 1 bis 4 Kohlenstoffatomen und 1 bis 5 Halogenatomen, gegebenenfalls durch Halogen substituiertes Cycloalkyl mit 3 bis 7 Kohlenstoffatomen, Naphthyl oder gegebenenfalls einfach bis dreifach, gleichartig oder verschieden durch Halogen, Nitro, Phenyl, Phenoxy, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen und/oder Halogenalkylthio mit 1 bis 4 Kohlenstoffatomen und 1 bis 5 Halogenatomen substituiertes Phenyl steht,
R² für gegebenenfalls einfach bis dreifach, gleichartig oder verschieden durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen und/oder Halogenalkylthio mit 1 bis 4 Kohlenstoffatomen und 1 bis 5 Halogenatomen substituiertes Phenyl steht und
R³ für Alkoxyalkyl, Isopropyl oder n-Dodecyl steht.

2. Oxiranyl-triazolinthione der Formel (I) bzw. (Ia) gemäß Anspruch 1, in welcher
R¹ für Methyl, Ethyl, n-Propyl, Isopropyl, n-Butyl, sek.-Butyl, iso-Butyl, tert.-Butyl, Fluor-tert.-butyl, Difluor-tert.-butyl, gegebenenfalls einfach bis dreifach, gleichartig oder verschieden durch Fluor, Chlor und/oder Brom substituiertes Cycloalkyl mit 3 bis 6 Kohlenstoffatomen, für Naphthyl oder für Phenyl steht, das einfach bis dreifach, gleichartig oder verschieden substituiert sein kann durch Fluor, Chlor, Brom, Nitro, Phenyl, Phenoxy, Methyl, Ethyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio,
R² für Phenyl steht, das einfach bis dreifach, gleichartig oder verschieden substituiert sein kann durch Fluor, Chlor, Brom, Methyl, Ethyl, Isopropyl, tert.-Butyl, Methoxy, Ethoxy, Trifluormethyl, Trichlormethyl, Difluormethyl, Difluorchlormethyl, Trifluormethoxy, Difluormethoxy und/oder Trifluormethylthio, und
R³ für Alkoxyalkyl mit 1 bis 6 Kohlenstoffatomen im Alkoxyteil und 1 bis 6 Kohlenstoffatomen im Alkylteil sowie für Isopropyl oder für n-Dodecyl steht.

3. Verfahren zur Herstellung von Oxiranyl-triazolinthionen der Formel (I) bzw. (Ia) gemäß Anspruch 1, **dadurch gekennzeichnet, daß** man Oxiran-Derivate der Formel 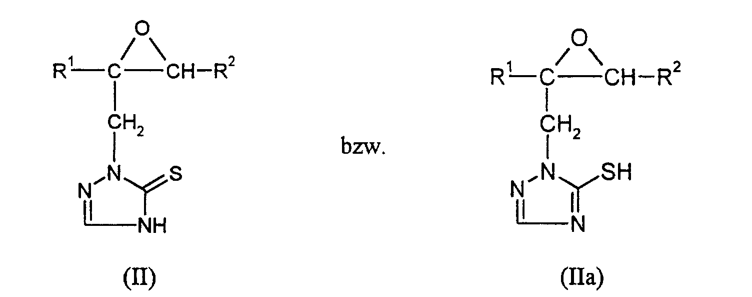 in welcher
R¹ und R² die oben angegebenen Bedeutungen haben,
mit Isocyanaten der Formel
R³-NCO (III),
in welcher
R³ die oben angegebene Bedeutung hat,
gegebenenfalls in Gegenwart eines Katalysators und gegebenenfalls in Gegenwart eines Verdünnungsmittels umsetzt.

4. Mikrobizide Mittel, **gekennzeichnet durch** einen Gehalt an mindestens einem Oxiranyl-triazolinthion der Formel (I) bzw. (Ia) gemäß Anspruch 1 neben Streckmitteln und/oder oberflächenaktiven Stoffen.

5. Verwendung von Oxiranyl-triazolinthionen der Formel (I) bzw. (Ia) gemäß Anspruch 1 als Mikrobizide im Pflanzenschutz und im Materialschutz.

6. Verfahren zur Bekämpfung von unerwünschten Mikroorganismen im Pflanzenschutz und im Materialschutz, **dadurch gekennzeichnet, daß** man Oxiranyl-triazolinthione der Formel (I) bzw. (Ia) gemäß Anspruch 1 auf die Mikroorganismen und/oder deren Lebensraum ausbringt.

7. Verfahren zur Herstellung von mikrobiziden Mitteln, **dadurch gekennzeichnet, daß** man Oxiranyl-triazolinthione der Formel (I) bzw. (Ia) gemäß Anspruch 1 mit Streckmitteln und/oder oberflächenaktiven Stoffen vermischt.

8. Oxiranyl-triazolinthion gemäß Anspruch 1, **gekennzeichnet durch** die Formel

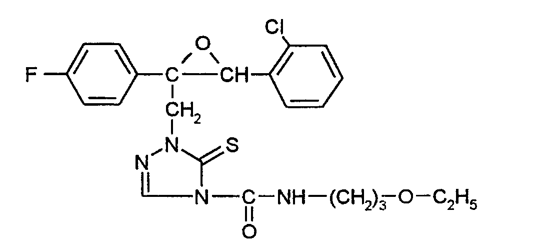

9. Oxiranyl-triazolinthion gemäß Anspruch 1, **gekennzeichnet durch** die Formel

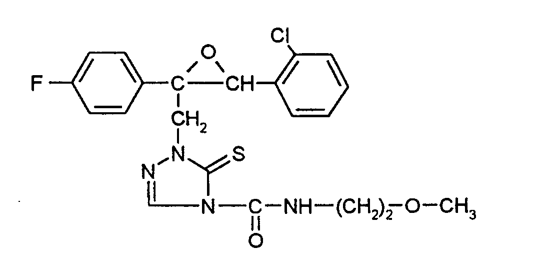

10. Oxiranyl-triazolinthion gemäß Anspruch 1, **gekennzeichnet durch** die Formel

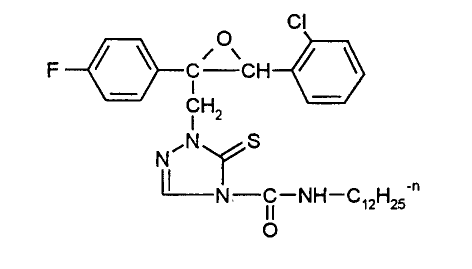

## Claims

1. Oxiranyl-triazolinethiones of the formula 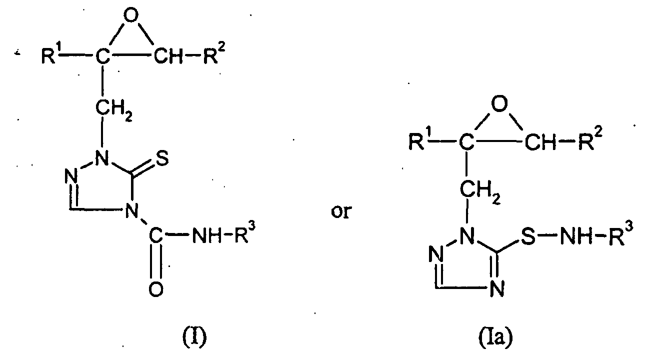 in which
R¹ represents alkyl having 1 to 4 carbon atoms, halogenoalkyl having 1 to 4 carbon atoms and 1 to 5 halogen atoms, optionally halogen-substituted cycloalkyl having 3 to 7 carbon atoms, naphthyl or phenyl which is optionally mono- to trisubstituted by identical or different substituents from the group consisting of halogen, nitro, phenyl, phenoxy, alkyl having 1 to 4 carbon atoms, alkoxy having 1 to 4 carbon atoms, halogenoalkyl having 1 to 4 carbon atoms and 1 to 5 halogen atoms, halogenoalkoxy having 1 to 4 carbon atoms and 1 to 5 halogen atoms and halogenoalkylthio having 1 to 4 carbon atoms and 1 to 5 halogen atoms,
R² represents phenyl which is optionally mono- to trisubstituted by identical or different substituents from the group consisting of halogen, alkyl having 1 to 4 carbon atoms, alkoxy having 1 to 4 carbon atoms, halogenoalkyl having 1 to 4 carbon atoms and 1 to 5 halogen atoms, halogenoalkoxy having 1 to 4 carbon atoms and 1 to 5 halogen atoms and halogenoalkylthio having 1 to 4 carbon atoms and 1 to 5 halogen atoms and
R³ represents alkoxyalkyl, isopropyl or n-dodecyl.

2. Oxiranyl-triazolinethiones of the formula (I) or (Ia) according to Claim 1 in which
R¹ represents methyl, ethyl, n-propyl, isopropyl, n-butyl, sec-butyl, iso-butyl, tert-butyl, fluoro-tert-butyl, difluoro-tert-butyl, cycloalkyl having three to six carbon atoms which is optionally mono- to trisubstituted by identical or different substituents from the group consisting of fluorine, chlorine and bromine, represents naphthyl or represents phenyl which may be mono- to trisubstituted by identical or different substituents from the group consisting of fluorine, chlorine, bromine, nitro, phenyl, phenoxy, methyl, ethyl, tert-butyl, methoxy, ethoxy, trifluoromethyl, trichloromethyl, difluoromethyl, difluorochloromethyl, trifluoromethoxy, difluoromethoxy and trifluoromethylthio,
R² represents phenyl which may be mono- to trisubstituted by identical or different substituents from the group consisting of fluorine, chlorine, bromine, methyl, ethyl, isopropyl, tert-butyl, methoxy, ethoxy, trifluoromethyl, trichloromethyl, difluoromethyl, difluorochloromethyl, trifluoromethoxy, difluoromethoxy and trifluoromethylthio, and
R³ represents alkoxyalkyl having 1 to 6 carbon atoms in the alkoxy moiety and 1 to 6 carbon atoms in the alkyl moiety and also represents isopropyl or n-dodecyl.

3. Process for preparing oxiranyl-triazolinethiones of the formula (I) or (Ia) according to Claim 1, **characterized in that** oxirane derivatives of the formula 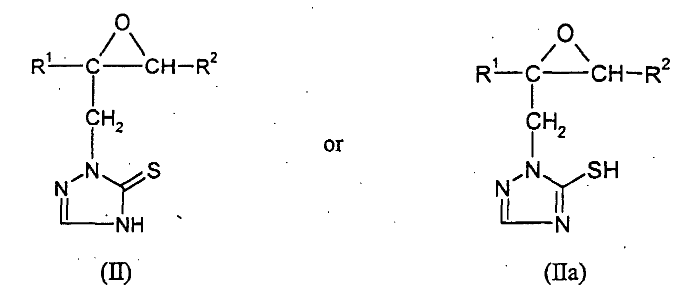 in which
R¹ and R² are each as defined above
are reacted with isocyanates of the formula
R³-NCO (III),
in which
R³ is as defined above,
if appropriate in the presence of a catalyst and if appropriate in the presence of a diluent.

4. Microbicidal compositions, **characterized in that** they comprise at least one oxiranyl-triazolinethione of the formula (I) or (Ia) according to Claim 1, in addition to extenders and/or surfactants.

5. Use of oxiranyl-triazolinethiones of the formula (I) or (Ia) according to Claim 1 as microbicides in crop protection and in the protection of materials.

6. Method for controlling undesirable microorganisms in crop protection and in the protection of materials, **characterized in that** oxiranyl-triazolinethiones of the formula (I) or (Ia) according to Claim 1 are applied to the microorganisms and/or their habitat.

7. Process for preparing microbicidal compositions, **characterized in that** the oxiranyl-triazolinethiones of the formula (I) or (Ia) according to Claim 1 are mixed with extenders and/or surfactants.

8. Oxiranyl-triazolinethione according to Claim 1, **characterized by** the formula

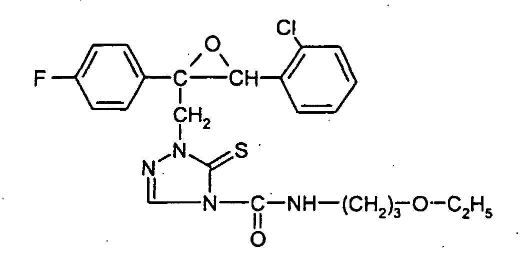

9. Oxiranyl-triazolinethione according to Claim 1, **characterized by** the formula

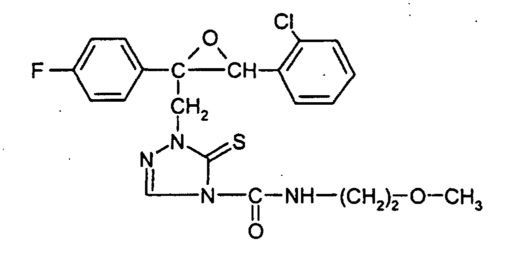

10. Oxiranyl-triazolinethione according to Claim 1, **characterized by** the formula

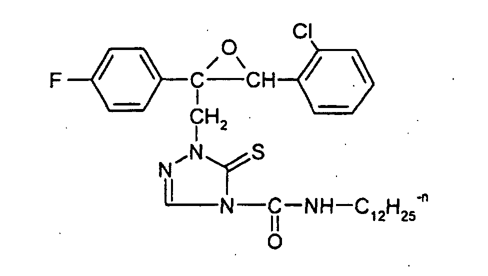

## Revendications

1. Oxiranyltriazoline-thiones de formule 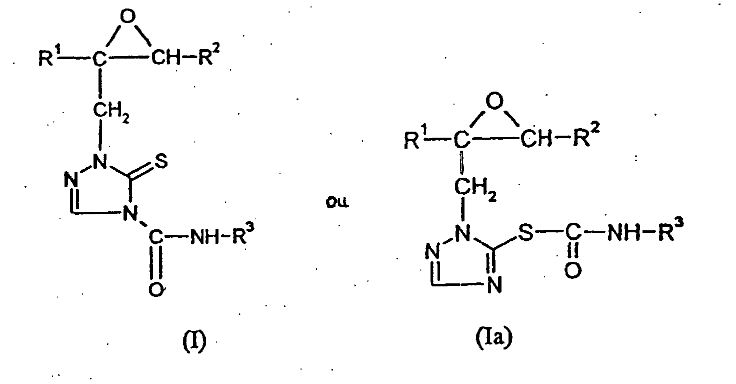 dans laquelle
R¹ est un reste alkyle·ayant 1 à 4 atomes de carbone, un reste halogénalkyle ayant 1 à 4 atomes de carbone et 1 à 5 atomes d'halogènes, un reste cycloalkyle de 3 à 7 atomes de carbone éventuellement substitué par un halogène, un reste naphtyle ou un reste phényle éventuellement substitué une à trois fois identiques ou différentes par un radical halogéno, nitro, phényle, phénoxy, alkyle ayant 1 à 4 atomes de carbone, alkoxy ayant 1 à 4 atomes de carbone, halogénalkyle ayant 1 à 4 atomes de carbone et 1 à 5 atomes d'halogènes, halogénalkoxy ayant 1 à 4 atomes de carbone et 1 à 5 atomes d'halogènes et/ou halogénalkylthio ayant 1 à 4 atomes de carbone et 1 à 5 atomes d'halogènes,
R² est un reste phényle éventuellement substitué une à trois fois identiques ou différentes par un radical halogéno, alkyle ayant 1 à 4 atomes de carbone, alkoxy ayant 1 à 4 atomes de carbone halogénalkyle ayant 1 à 4 atomes de carbone et 1 à 5 atomes d'halogènes, halogénalkoxy ayant 1 à 4 atomes de carbone et 1 à 5 atomes d'halogènes et/ou halogénalkylthio ayant 1 à 4 atomes de carbone et 1 à 5 atomes d'halogènes, et
R³ est un reste alkoxyalkyle, isopropyle ou n-dodécyle.

2. Oxiranyltriazoline-thiones de formule (I) ou (Ia) suivant la revendication 1, formule dans laquelle
R¹ est un reste méthyle, éthyle, n-propyle, isopropyle, n-butyle, sec.-butyle, isobutyle, tertiobutyle, fluorotertiobutyle, difluorotertiobutyle, un reste cycloalkyle ayant 3 à 6 atomes de carbone éventuellement substitué une à trois fois identiques ou différentes par du fluor, du chlore et/ou du brome, un reste naphtyle ou un reste phényle qui peut être substitué une à trois fois identiques où différentes par du fluor, du chlore, du brome, un radical nitro, phényle, phénoxy, méthyle, éthyle, tertiobutyle, méthoxy, éthoxy, trifluorométhyle, trichlorométhyle, difluorométhyle, difluorochlorométhyle, trifluorométhoxy, difluorométhoxy, et/ou trifluorométhylthio,
R² est un reste phényle qui peut être substitué une à trois fois identiques ou différentes par du fluor, du chlore, du brome, un radical méthyle, éthyle, isopropyle, tertiobutyle, méthoxy, éthoxy, trifluorométhyle, trichlorométhyle, difluorométhyle, difluorochlorométhyle, trifluorométhoxy, difluorométhoxy et/ou trifluorométhylthio, et
R³ est un reste alkoxyalkyle ayant 1 à 6 atomes de carbone dans la partie alkoxy et 1 à 6 atomes de carbone dans la partie alkyle, ainsi qu'un reste isopropyle ou un reste n-dodécyle.

3. Procédé de production d'oxiranyltriazolinethiones de formule (I) ou (Ia) suivant la revendication 1, **caractérisé en ce qu'**on fait réagir des dérivés d'oxirane de formule 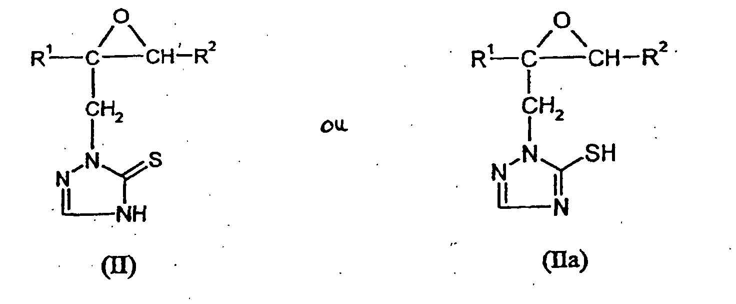 dans laquelle
R¹ et R² ont les définitions indiquées ci-dessus,
avec des isocyanates de formule
R³-NCO (III),
dans laquelle
R³ a la définition indiquée ci-dessus,
éventuellement en présence d'un catalyseur et, le cas échéant, en présence d'un diluant.

4. Compositions microbicides, **caractérisées par** une teneur en au moins une oxiranyltriazoline-thione de formule (I) ou (Ia) suivant la revendication 1, à côté de diluants et/ou d'agents tensioactifs.

5. Utilisation d'oxiranyltriazoline-thiones de formule (I) ou (Ia) suivant la revendication 1 comme microbicides dans la protection des plantes et dans la protection des matériaux.

6. Procédé de lutte contre des microorganismes indésirables dans la protection des plantes et dans la protection des matériaux, **caractérisé en ce qu'**on épand des oxiranyltriazoline-thiones de formule (I) ou (Ia) suivant la revendication 1 sur les microorganismes et/ou sur leur milieu.

7. Procédé de préparation de compositions microbicides, **caractérisé en ce qu'**on mélange des oxiranyltriazoline-thiones de formule (I) ou (Ia) suivant la revendication 1, avec des diluants et/ou des agents tensioactifs.

8. Oxiranyltriazoline-thione suivant la revendication 1, **caractérisée par** la formule

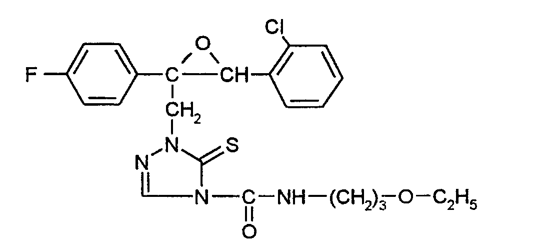

9. Oxiranyltriazoline-thione suivant la revendication 1, **caractérisée par** la formule

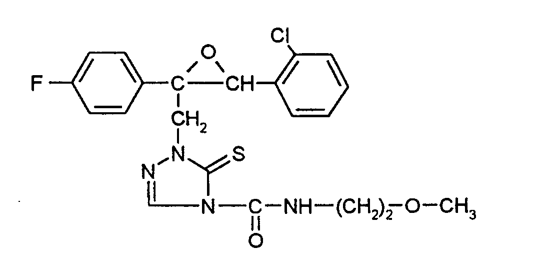

10. Oxiranyltriazoline-thione suivant la revendication 1, **caractérisée par** la formule
